# EUROPEAN PATENT SPECIFICATION

(11) **EP 4 043 566 B1**
(45) Date of publication and mention of the grant of the patent: **10.04.2024**
(21) Application number: 21156992.6
(22) Date of filing: 12.02.2021
(51) Int. Cl.: C12N 15/113, C12Q 1/68, C12N 15/115

(54) **DEOXYRIBOZYMES GENERATING CHEMILUMINESCENT SIGNALS**
DESOXYRIBOZYME ZUR ERZEUGUNG VON CHEMILUMINESZENTEN SIGNALEN
DEOXYRIBOZYMES GÉNÉRANT DES SIGNAUX CHIMILUMINESCENTS

(43) Date of publication of application: 17.08.2022
(73) Proprietor: Ustav organicke chemie a biochemie AV CR, v.v.i., 166 10 Praha 6 (CZ)
(72) Inventor: Curtis, Edward Arthur, Fullerton, California 93835 (US); Svehlova, Katerina, 17000 Praha 7 (CZ); Jakubec, Martin, 92101 Banka (SK); Luksan, Ondrej, 19800 Praha 9 (CZ)
(74) Representative: Hartvichova, Katerina

(56) References cited:
- WO-A2-2005/051174
- WO-A2-2008/127382
- SHAHSAVAR KOSAR ET AL: "A sensitive colorimetric aptasensor with a triple-helix molecular switch based on peroxidase-like activity of a DNAzyme for ATP detection", ANALYTICAL METHODS, vol. 9, no. 32, 1 January 2017 (2017-01-01), pages 4726-4731, XP055822357, GB ISSN: 1759-9660, DOI: 10.1039/C7AY01381G
- EUN JEONG CHO ET AL: "Applications of Aptamers as Sensors", ANNUAL REVIEW OF ANALYTICAL CHEMISTRY, vol. 2, no. 1, 19 July 2009 (2009-07-19), pages 241-264, XP055036844, ISSN: 1936-1327, DOI: 10.1146/annurev.anchem.1.031207.112851
- BRONSTEIN I ET AL: "1,2-DIOXETANES: NOVEL CHEMILUMINESCENT ENZYME SUBSTRATES. APPLICATIONS TO IMMUNOASSAYS", JOURNAL OF BIOLUMINESCENCE AND CHEMILUMINESCENCE, vol. 4, no. 1, 1 July 1989 (1989-07-01), pages 99-111, XP008102186, ISSN: 0884-3996, DOI: 10.1002/BIO.1170040116 [retrieved on 2005-04-09]
- ZHOU ZHAOJUAN ET AL: "A general approach for rational design of fluorescent DNA aptazyme sensors based on target-induced unfolding of DNA hairpins", ANALYTICA CHIMICA ACTA, vol. 889, 7 August 2015 (2015-08-07), pages 179-186, XP029273371, ISSN: 0003-2670, DOI: 10.1016/J.ACA.2015.06.036

## Description

### Field of Art

The present invention relates to deoxyribozymes and their uses.

### Background Art

Once thought to function primarily as a passive carrier of genetic information, RNA and DNA molecules are now known to be capable of a wide range of functions. For example, nucleic acid molecules called ribozymes (RNA) or deoxyribozymes (DNA) can act as catalysts which accelerate reaction rates millions of times relative to the corresponding nonenzymatic reactions. The structures and functions of catalytic nucleic acids can be allosterically modulated by ligands, and this has been utilized to construct sensors made of RNA and DNA (Tang,J. and Breaker,R.R. (1997) Chem Biol, 4, 453-459; Koizumi,M., Soukup,G.A., Kerr,J.N. and Breaker,R.R. (1999) Nat Struct Biol, 6, 1062-1071). Such sensors consist of a binding domain, which binds the ligand, and a signaling domain, which produces a detectable signal only in the presence of the ligand. Early examples used a self-cleaving ribozyme, and reaction products were detected by PAGE. Later experiments used chemically modified RNA substrates containing a fluorophore at one end and a quencher at the other. Ribozyme-catalyzed cleavage separated the fluorophore from the quencher, and generated a fluorescent signal that could be detected in solution and monitored in real time. Several additional signaling domains made of DNA or RNA have recently been described. These include G-quadruplexes with peroxidase activity which can generate colorimetric or chemiluminescent readouts (Kosman,J. and Juskowiak,B. (2011) Anal Chim Acta, 707, 7-17) and RNA aptamers which enhance the fluorescence of ligands to which they bind (Paige,J.S., Wu,K.Y. and Jaffrey,S.R. (2011) Science, 333, 642-646). These domains typically generate signals 10 to 2000-fold higher than background levels. WO-A-2005/051174 describes aptazymes for detecting biohasardous entities by using 1,2-dioxetanes as chemiluminescent substrates.

Limitations include the requirement for expensive chemically modified oligonucleotides or fluorophores that are not commercially available.

### Disclosure of the Invention

The invention relates to a deoxyribozyme which catalyzes a self-phosphorylation reaction in which a phosphate group is transferred from a substrate to the deoxyribozyme. The phosphorylation reaction thus involves cleavage of the phosphate group from the substrate, and its transfer to the deoxyribozyme. The invention further relates to methods of detection of the substrate, or of the deoxyribozyme, or of a ligand such as an oligonucleotide.

The substrate is a compound containing a phosphate functional group and shall be configured so that the cleavage of the phosphate functional group causes a measurable optical signal to be generated. The optical signal may be within the visible light wavelength range, or outside the visible light wavelength range, e.g., in UV or IR wavelengths. Such substrates may be described as chemiluminescent.

The chemiluminescent substrate is preferably a compound of the group of phosphate-stabilized 1,2-dioxetanes. Such substrates are described, e.g., in Bronstein,I., Edwards,B. and Voyta,J.C. (1989) J Biolumin Chemilumin, 4, 99-111; Trayhum,P., Thomas,M.E., Duncan,J.S., Black,D., Beattie,J.H. and Rayner,D.V. (1995) Biochem Soc Trans, 23, 494S. The compound disclosed in the cited prior art as phosphate-stabilized 1,2-dioxetanes or as chemiluminescent substrates are incorporated herein as examples of chemiluminescent substrates.

1,2-dioxetanes are 4-member ring peroxides which decompose (in the case of our substrates, after cleavage of the stabilizing phosphate group) to form products which are in an excited electronic state. These products may decompose further and emit light.

An example of a chemiluminescent substrate is disodium 2-chloro-5-(4-methoxyspiro{1,2-dioxetane-3,2'-(5'-chloro)tricyclo[3.3.1.1^{3.7}]decan}-4-yl)phenyl phosphate (commercially available under the trade name CDP-Star^{™}). The CDP-Star substrate is known as a substrate of alkaline phosphatase for use in detection of alkaline phosphatase or alkaline phosphatase conjugates, and for detection of non-radioactively labeled nucleic acids in Southern blots, Northern blots, dot blots, colony or plaque hybridizations, gel shift assays. Upon dephosphorylation, the CDP-Star substrate forms meta-stable dioetane phenolate anion which decomposes and emits light at 466 nm.

Another example of a chemiluminescent substrate is disodium 3-(4-methoxyspiro {1,2-dioxetane-3,2'-(5'-chloro)tricyclo[3.3.1.13.7]decan}-4-yl) phenyl phosphate (commercially available under the trade name CSPD^{™}). The chemical structure is similar to CDP-Star, and it is used for similar applications.

The deoxyribozyme of the present invention contains or consists of the following nucleotide sequence: 
5′-X¹X²X³AX⁴X⁵-R¹-X⁶X⁷X⁸X⁹X¹⁰-bp1-helix1-R²-helix2-bp2-X¹¹TGACX¹²TGGGAX¹³-helix3-X¹⁴-
R³-3′ (SEQ ID NO. 1)
 wherein
X¹ is G or T; X² is G or T; X³ is A or C; X⁴ is G or T; X⁵ is A or T; X⁶ is G or T or A; X⁷ is A or C or G or T; X⁸ is A or G or T; X⁹ is T or C or A or G; X¹⁰ is A or T or G; X¹¹ is G or A; X¹² is T or C or G; X¹³ is T or G or C; X¹⁴ is G or A or C or T;
R¹ represents a nucleotide sequence containing 3 to 30 nucleotides;
R² represents a nucleotide sequence containing 3 to 30 nucleotides;
R³ represents a nucleotide sequence containing 0 to 30 nucleotides;
bp1 and bp2 are nucleotides selected from G, A, C, T, which together form a base pair;
helix1, helix2, and helix3 each independently denotes a nucleotide sequence preferably containing 4 or more nucleotides, wherein helix1, helix2 and helix3 together form a triple helical structure.

In some embodiments, the deoxyribozyme of the present invention contains or consists of the following nucleotide sequence: 
5′-X¹X²X³AX⁴X⁵-R¹-X⁶X⁷X⁸X⁹X¹⁰-bp1-helix1-R²-helix2-bp2-X¹¹TGACX¹²TGGGAX¹³-helix3-X¹⁴-
R³-3′ (SEQ ID NO. 1)
 wherein
X¹ is G; X² is G; X³ is A or C; X⁴ is G; X⁵ is A or T; X⁶ is G; X⁷ is A; X⁸ is A; X⁹ is Tor C; X¹⁰ is A; X¹¹ is G; X¹² is T or C; X¹³ is T; X¹⁴ is G or A or C or T;
and wherein one or more, preferably one or two, of X¹, X², X⁴, X⁶-X¹³ are replaced by the following nucleotides: X¹ = T; X² = T; X⁴ = T; X⁶ = T or A; X⁷ = C or G or T; X⁸ = G or T; X⁹ = A or G; X¹⁰ = T or G; X¹¹ = A; X¹² = G; X¹³ = G or C;
R¹ represents a nucleotide sequence containing 3 to 30 nucleotides;
R² represents a nucleotide sequence containing 3 to 30 nucleotides;
R³ represents a nucleotide sequence containing 0 to 30 nucleotides;
bp1 and bp2 are nucleotides selected from G, A, C, T, which together form a base pair;
helix1, helix2 and helix3 each independently denotes a nucleotide sequence preferably containing 4 or more nucleotides, wherein helix 1 , helix2, and helix3 together form a triple helical structure.

In some embodiments, the deoxyribozyme of the present invention contains or consists of the following nucleotide sequence:
5'-GGX³AGX⁵-R¹-GAAX⁹A-bpl-helixl-R²-helix2-bp2-GTGACX¹²TGGGAT-helix3-X¹⁴-R³-3' (SEQ ID NO. 2)
wherein
X³ is A or C; X⁵ is A or T; X⁹ is T or C; X¹² is Tor C; X¹⁴ is G or A or C or T;
R¹ represents a nucleotide sequence containing 3 to 30 nucleotides;
R² represents a nucleotide sequence containing 3 to 30 nucleotides;
R³ represents a nucleotide sequence containing 0 to 30 nucleotides;
bp1 and bp2 are nucleotides selected from G, A, C, T, which together form a base pair;
helix 1 , helix2, and helix3 each independently denotes a nucleotide sequence preferably containing 4 or more nucleotides, wherein helix 1 , helix2, and helix3 together form a triple helical structure.

In some embodiments, the deoxyribozyme contains or consists of the following nucleotide sequence: 5'-GGAAGA-R¹-GAATA-bp1-helix1-R²-helix2-bp2-GTGACTTGGGAT-helix3-G-R³-3' (SEQ ID NO. 3), wherein
R¹ represents a nucleotide sequence containing 3 to 30 nucleotides;
R² represents a nucleotide sequence containing 3 to 30 nucleotides;
R³ represents a nucleotide sequence containing 0 to 30 nucleotides;
bp1 and bp2 are nucleotides selected from G, A, C, T, which together form a base pair;
helix1, helix2, and helix3 each independently denotes a nucleotide sequence preferably containing 4 or more nucleotides, wherein helix 1 , helix2, and helix3 together form a triple helical structure.

Generally, nucleotides are selected from adenine nucleotide (A), cytosine nucleotide (C), guanine nucleotide (G), and thymine nucleotide (T). A nucleotide generally contains a base (adenine or cytosine or guanine or thymine) covalently linked to a deoxyribose which is in turn covalently linked to a phosphate.

Preferably, R¹ contains 3 to 30 nucleotides, more preferably 3 to 4 nucleotides.

In some embodiments, R¹ is a poly-A sequence.

Preferably, R² contains 3 to 30, more preferably 3 to 10 nucleotides.

In some embodiments, R² is a poly-A sequence. In some embodiments, R² is or contains an aptamer or a sequence complementary to an aptamer.

Preferably, R³ contains 3 to 30, more preferably 3 to 10 nucleotides.

In some embodiments, R³ is a poly-A sequence. In some embodiments, R³ is or contains an aptamer or a sequence complementary to an aptamer.

In some embodiments, when R² is or contains an aptamer, then R³ is or contains a sequence complementary to the aptamer, and when R³ is or contains an aptamer, then R² is or contains a sequence complementary to the aptamer.

An aptamer is an oligonucleotide sequence that binds to a specific target molecule or target sequence (target molecules and target sequences fall into the group called herein more generally "ligands"). Many aptamer sequences for specific target molecules are known in the art (for example in: Gold,L., Polisky,B., Uhlenbeck,O. and Yarus,M. (1995) Annu Rev Biochem, 64, 763-797; Cho,E.J., Lee,J.W. and Ellington,A.D. (2009) Annu Rev Anal Chem, 2, 241-264). An aptamer for a target sequence is a complementary sequence.

The base pair bp1 and bp2 is preferably formed by canonical T-A, A-T, C-G, and G-C base pairs, more preferably by T-A and A-T base pairs.

The triple helical structure is preferably formed by combinations of canonical C-G:G, T-A:A, and TA:T base triples, more preferably by C-G:G base triples.

Preferably, each of the helixes 1-3 has the length of 4-10 nucleotides, more preferably 4-7 nucleotides. In some embodiments, helix 1, helix 2, helix 3 is CCCC, GGGG, GGGG or CCCT, AGGG, GGGT or CTCC, GGAG, GAGG or CCCT, AGGG, GGGA.

In one preferred embodiment, helix1 has the sequence of CCCC, helix 2 has the sequence GGGG, and helix 3 has the sequence GGGG.

Partially disrupted triples can also occur at some positions. These have the ability to form either the Watson-Crick part of the base triple (represented by "-") or the Hoogsteen part of the base triple (represented by ":").

When each of the helices 1-3 has the length of 4 nucleotides, they form four triples.

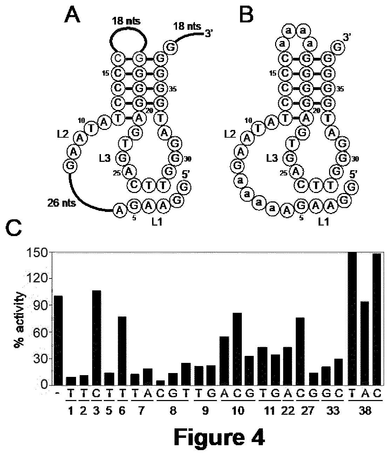

The first triple (positions 13-20-34 in the minimized catalytic core; see Figure 4 for the numbering scheme) may be selected from A G:G, T G:G, A A:T, G G:G, A A:A, G A:A, G A:T, and C A:A, preferably A G:G and T G:G.

The second triple (positions 14-19-35 in the minimized catalytic core; see Figure 4 for the numbering scheme) may be selected from C-G T, G A:A, C-G A, A G:G, T G:G, A A:A, G A:T, A A:T, C A:A, and G G:G, preferably C-G T and G A:A.

The third triple (positions 15-18-36 in the minimized catalytic core; see Figure 4 for the numbering scheme) may be selected from G G:G, T G:G, A A:T, G A:T, C-G T, A A:A, A G:G, C-G A, C A:A, and T-A G, preferably G G:G and T G:G.

The fourth triple (positions 16-17-37 in the minimized catalytic core; see Figure 4 for the numbering scheme) may be selected from C A:T, A A:T, A G:G, A-T A, G A:T, T G:G, C-G T, A A:A, C-G A, C A:A, G-C A, T G T, G G:G, T-A G, G A:A, G T A, G G C, and C A G, preferably C A:T and A A:T.

Preferably, the deoxyribozyme contains 45 to 160 nucleotides, more preferably 45 to 110 nucleotides in total, even more preferably 80 to 110 nucleotides.

The invention thus provides a deoxyribozyme that phosphorylates itself in the presence of a phosphate group-containing substrate, such as phosphate-stabilized 1,2-dioxetane substrates, to generate an optical signal. By "deoxyribozyme", it is meant a DNA oligonucleotide which catalyzes a chemical reaction. This meaning includes DNA oligonucleotides with chemical modifications. Deoxyribozymes of the present invention were developed with the aid of a method of artificial evolution called *in vitro* selection. Catalytic oligonucleotides isolated using *in vitro* selection typically contain a catalytic core, usually consisting of stems formed by A-T, T-A, G-C, and C-G base pairs, interspersed with sequence elements not required for function.

The catalytic core of the deoxyribozyme described herein has the following features:
- L1 (i.e., the sequence X¹X²X³AX⁴X⁵) occurs at the 5' end of the deoxyribozyme. The consensus sequence of L1 is GGAAGA. Of the 50 most abundant deoxyribozyme variants identified (which make up 5.1 million of the 7.1 million total reads generated using high-throughput sequencing), 94.2% contain GGAAGA here. Other possibilities include GGCAGA (3.2% of these reads), and GGAAGT (2.6% of these reads).
- L1 is connected to L2 (i.e., the sequence X⁶X⁷X⁸X⁹X¹⁰) by a spacer R¹. The spacer may contain any combination of nucleotides selected from A, C, G, and T. A typical length of the spacer is from 3 to 30 nucleotides. 23,186 different variants of R¹ were identified among the 135,000 different deoxyribozyme variants we identified (in this case R¹ contained 26 nucleotides).
- The consensus sequence of L2 (i.e., the sequence X⁶X⁷X⁸X⁹X¹⁰) is GAATA. Of the 50 most abundant deoxyribozyme variants identified (which make up 5.1 million of the 7.1 million total reads), 83.2% contain GAATA here. Other possibilities include GCATA (8.4% of these reads), GAATT (5.7% of these reads), and GTATA (0.8% of these reads).
- L2 is connected at its 3' end to a base pair which may consist of T-A, A-T, C-G, or G-C.
- The 3' end of the 5' position in the base pair is connected to a triple helix.
- The triple helix formed from helix1, helix2, and helix3 may contain various combinations of canonical C-G:G, T-A:A, and T-A:T triples. Optionally other nucleotide triples may be present, as long as the triple-helical structure is maintained.
- The triple helix is capped by a loop R² with a sequence N₃₋₃₀ (N = A, C, G or T). 18,024 different variants of R² were identified among the 135,000 different deoxyribozyme variants we identified (in this case R² contained 18 nucleotides).
- L3 (i.e., the sequence X¹¹TGACX¹²TGGGAX¹³) connects the 3' end of helix2 to helix3. The consensus sequence of L3 is GTGACTTGGGAT. Of the 50 most abundant deoxyribozyme variants identified (which make up 5.1 million of the 7.1 million total reads), 100% contain GTGACTTGGGAT here.
- The 3' end of the deoxyribozyme contains a single nucleotide overhang (X¹⁴). Of the 50 most abundant deoxyribozymes variants identified (which make up 5.1 million of the 7.1 million total reads), 86% contain G here. Other possibilities include T (12% of these reads), and A (2% of these reads).

This overhang can be extended by the extension R³ with the sequence N₀₋₃₀ (N = A, C, G, or T).

The deoxyribozyme of the present invention, upon contact with a substrate, can cause the generation of an optical signal. The optical signal is generated by dephosphorylation of the substrate by the deoxyribozyme. This triggers decomposition of the substrate and, in case of the CDP-Star^{™} and CSPD^{™} substrates, production of blue light with a wavelength of ~466 nm by a CIEEL mechanism. The deoxyribozyme can be configured so that it is inactive (inactive state) in the absence of a ligand, but undergoes an allosteric change to the catalytically active structure (active state) in the presence of the ligand. In this way the deoxyribozyme can be used to detect specific ligands. The substrate is as defined herein above.

The present invention further relates to a method of *in vitro* detection of a chemiluminescent substrate, containing a phosphate functional group, in a sample, containing the steps of:
- bringing the sample into contact with the deoxyribozyme of the present invention;
- causing the generation of an optical signal by cleavage of the phosphate functional group from the substrate present in the sample by self-phosphorylation of the deoxyribozyme;
- detecting the generated optical signal;
- optionally detecting the amount of the generated optical signal.

In this method, it is advantageous when R1 and R2 are not mutually complementary.

In this method, it is advantageous when R1 and R3 are not mutually complementary.

In this method, it is advantageous when R2 and R3 are not mutually complementary.

The present invention also relates to a method of *in vitro* detection of the deoxyribozyme in a sample, containing the steps of:
- bringing the sample into contact with a chemiluminescent substrate containing a phosphate functional group;
- causing the generation of an optical signal by cleavage of the phosphate functional group from the substrate by self-phosphorylation of the deoxyribozyme present in the sample;
- detecting the generated optical signal;
- optionally detecting the amount of the generated optical signal.

The step of causing the generation of the optical signal typically includes providing suitable reaction conditions for the self-phosphorylation reaction of the deoxyribozyme. Such reaction conditions may include, for example, metal ions in the buffer, including potassium, zinc, cerium, lead (e.g., 200 mM KCl, 1 mM ZnCl₂,1 µM Ce(SO₄)₂, and 0.1 µM PbCh), pH (e.g. 7-8), buffer (e.g. HEPES), temperature (e.g. 20-30 °C), deoxyribozyme concentration (e.g. 0.1-10 µM), and/or substrate concentration (e.g. 0.001-1 mM).

The present invention further relates to a method of *in vitro* detection of a ligand in a sample, containing the steps of:
- bringing the sample into contact with the deoxyribozyme of the present invention and with a substrate containing a phosphate functional group and capable of generating an optical signal upon cleavage of the phosphate functional group; wherein the deoxyribozyme is in an inactive state (induced by an inhibitory helix formed by base pairing between a sequence inserted into R² and R³) and wherein R³ contains an aptamer for the ligand so that when the ligand binds to the aptamer, the deoxyribozyme is converted into an active state,;
- causing the generation of an optical signal by cleavage of the phosphate functional group from the substrate present in the sample by self-phosphorylation of the deoxyribozyme in the active state;
- detecting the generated optical signal;
- optionally detecting the amount of the generated optical signal.

The ligand may be, for example, an oligonucleotide, a small molecule (e.g., molecular weight up to 2000 g/mol), a metal ion, a protein, or a lipid. The ligand is in fact an analyte detected in a sample.

The ligand binds to the aptamer, thus changing the conformation of the deoxyribozyme from the inactive state to the active state, and the deoxyribozyme in the active state then self-phosphorylates, thus cleaving the phosphate group from the substrate. This triggers a decomposition reaction and the production of blue light by a CIEEL mechanism.

For example, when the ligand is an oligonucleotide, the R² in the deoxyribozyme may be or contain the sequence or part of the sequence of the ligand (oligonucleotide), and the R³ in the doxyribozyme may be or contain a sequence complementary to the sequence or to part of the sequence of the ligand. The oligonucleotide binds to R³ by Watson-Crick pairing. At sufficiently high concentrations, this prevents formation of the inhibitory interaction between R² and R³. When the oligonucleotide is bound to R³, the deoxyribozyme is catalytically active and transfers the phosphate group from the substrate to the deoxyribozyme. This triggers a decomposition reaction and the production of blue light by a CIEEL mechanism.

Also when the ligand is a small molecule or a metal ion, or a protein, or a lipid, binding of the ligand to an aptamer suitable for the ligand causes conformational changes in the deoxyribozyme leading to its self-phosphorylation. For example, a sequence complementary to the aptamer can be inserted into R² and the aptamer can be inserted into R³. In the absence of the ligand of the aptamer, an inhibitory interaction will form between R² and R³ by Watson-Crick pairing. In the presence of sufficiently high concentrations of the ligand, however, the aptamer will bind the ligand rather than R². This will prevent formation of the inhibitory interaction between R² and R³. When the ligand is bound to the aptamer, the deoxyribozyme is catalytically active and transfers the phosphate group from the substrate to the deoxyribozyme. This triggers a decomposition reaction and the production of blue light by a CIEEL mechanism.

The step of causing the generation of the optical signal typically includes providing suitable reaction conditions for the self-phosphorylation reaction of the deoxyribozyme. Such reaction conditions may include, for example, metal ions in the buffer, including potassium, zinc, cerium, lead (e.g., 200 mM KCl, 1 mM ZnCl₂, 1 µM Ce(SO₄)₂, and 0.1 µM PbCh), pH (e.g. 7-8), buffer (e.g. HEPES), temperature (e.g. 20-30 °C), deoxyribozyme concentration (e.g. 0.1-10 µM), and/or substrate concentration (e.g. 0.001-1 mM), and ligand concentration (e.g. 1 to 100 µM).

Light generated by the deoxyribozyme can be detected using an instrument such as a plate reader, e.g. Tecan Spark plate reader.

Generally, the detection of the light may be qualitative or quantitative. Quantitative detection contains an additional step of determining the amount of the optical signal generated.

The invention also relates to the use of the deoxyribozyme of the present invention as a signaling component of molecular devices in applications such as biosensing and molecular computing. Molecular devices may include molecular sensors or molecular switches, such as deoxyribozymes that only generate light in the presence of a specific ligand such as ATP. Suitable applications of such molecular devices include diagnostics, high-throughput screens, and molecular computing. The deoxyribozyme of the present invention is used in the molecular device as the signaling component causing the generation of an optical signal which indicates the presence of a ligand (e.g., a metal ion, a small molecule, a protein, an oligonucleotide, a lipid), and optionally the molecular device is configured to perform an action upon generation of the said signal.

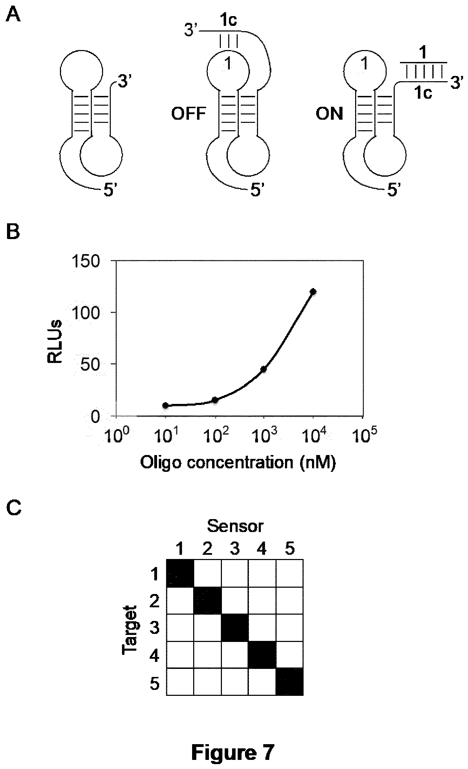

A second object of the invention relates to the use of this deoxyribozyme in molecular devices that generate a chemiluminescent signal. An example of such a device is an allosterically regulated sensor in which the deoxyribozyme described here (the signaling domain) is covalently linked to an aptamer (the binding domain) that binds a small molecule, oligonucleotide, or protein with high affinity and specificity. An example of such a sensor is shown in Figure 7 and Example 15. The deoxyribozyme contains an oligonucleotide binding site, and the amount of light produced is correlated with the concentration of the oligonucleotide in the buffer from 100 nM to 10 µM. Such a sensor can be constructed to detect any ligand, based on the information available in the art and based on the knowledge of a person skilled in the art.

### Figures

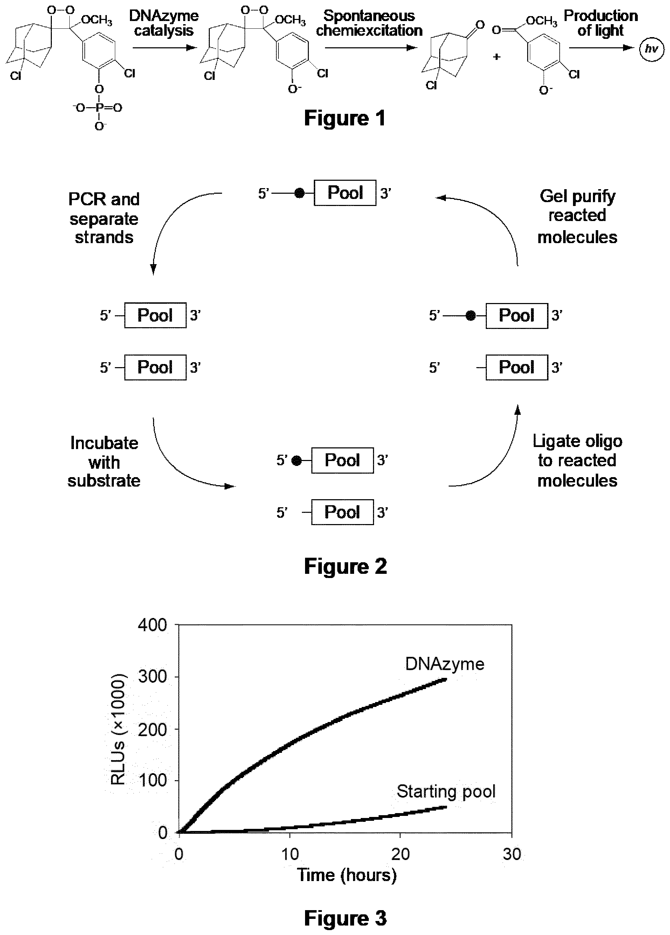

FIG. 1: Reaction scheme of deoxyribozyme-catalyzed light production using the CDP-Star^{™} substrate.
FIG. 2: Schematic diagram of the method used to develop deoxyribozymes that phosphorylate themselves in the presence of the chemiluminescent substrate as described in Example 1.
FIG. 3: Light production of a deoxyribozyme in the presence of the chemiluminescent CDP-Star^{™} substrate (see also Example 4).
FIG. 4: Minimized catalytic core and preferred nucleotides at unpaired positions. (A) Deoxyribozyme before minimization (see also Example 2). (B) Deoxyribozyme after minimization (see also Example 7). Solid horizontal lines = hydrogen bonds in base pairs in the Watson-Crick duplex of the triple helix; dashed horizontal lines = hydrogen bonds formed by the third strand of the triple helix. Note that only positions in the minimized catalytic core of the deoxyribozyme (shown in capital letters) are numbered. (C) Effects of mutations in unpaired positions. Only mutations with enrichment values ≥ 3.1×10⁻³ (frequency in the evolved pool divided by frequency in the starting pool) are shown. Percent activity is relative to SEQ ID NO:15 (shown in panel B and indicated by a dash in the graph). See also Example 9 and Table 2.

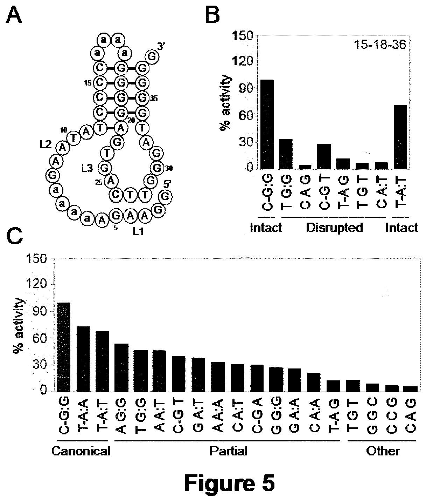

FIG. 5: Secondary structure model and analysis of base triples. (A) Secondary structure model. (B) Triple-mutant cycle consistent with the 15-18-36 base triple. See also Example 8. (C) Activity of canonical, partially disrupted ("partial"), and fully disrupted ("other") triples. Each triple was inserted into SEQ ID NO:15 (shown in panel A) at either 13-20-34, 14-19-35, 15-18-36, or 16-17-37; the average activity of these four mutants is indicated by each bar. See also Example 11 and Table 4.

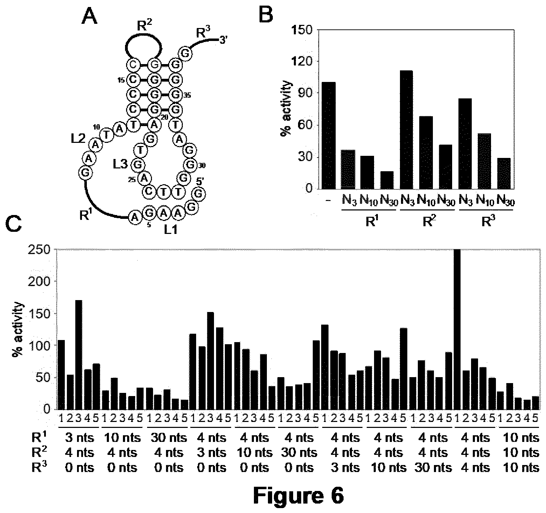

FIG. 6: (A) Secondary structure model showing positions (R', R², and R³) at which spacers with different lengths and sequences can be inserted. (B) Activity of deoxyribozymes containing randomized spacers of different lengths at R¹, R², or R³. N indicates a position containing 25% A, 25% C, 25% G, and 25% T. See also Example 13. (C) Activity of deoxyribozymes in which randomly chosen spacers of different lengths and sequences were inserted into R¹, R², and/or R³. Five different examples were tested for each architecture. See also Example 14.
FIG. 7: Deoxyribozyme sensor that produces light in the presence of a specific oligonucleotide, Example 5. (A) Design of the sensor. Left: deoxyribozyme before engineering. Middle: engineered deoxyribozyme sensor in the OFF conformation. 1 and 1c represent complementary sequences. Right: engineered deoxyribozyme sensor in the ON conformation in which the ligand (in this case an oligonucleotide) is bound to 1c by base pairing. (B) Sensitivity of the sensor. Production of light as a function of DNA concentration for an oligonucleotide complementary to the deoxyribozyme sensor. (C) Specificity of the sensor. Light production of five deoxyribozyme sensors (Example 15) in the presence of either an oligonucleotide complementary to the sensor (i.e. sensor 1 with target 1) or oligonucleotides not complementary to the sensor (i.e. sensor 1 with targets 2, 3, 4, or 5). Black squares indicate pairs for which the rate enhancement (light produced in the presence of target divided by light produced in the absence of target) exceeds 5-fold.

### Examples

### Example 1: Procedure for selecting light-producing deoxyribozymes

### Initial Selection

A single-stranded pool with the sequence GGAAGAGATGGCGACN₇₀AGCTGATCCTGATGG (SEQ ID NO. 4) was ordered from IDT and purified by PAGE. 10¹⁶ different sequences from this pool were mixed with the blocking oligonucleotide BO1 (CCATCAGGATCAGCT, SEQ ID NO. 5) in water, heated at 65 °C for 2 minutes and cooled at RT for 5 minutes. They were then incubated for 24 hours (round 1 - 6) or 10 minutes (round 7 - 9) with the substrate CDP-Star (Roche) as follows: 1 µM DNA pool, 1.5 µM blocking oligonucleotide BO1, 1× selection buffer (50 mM HEPES pH 7.4, 200 mM KCl, 1 mM ZnCl₂, 1 µM Ce(SO₄)₂, 0.1 µM PbCl₂), and 1 mM CDP-Star. DNA was then precipitated in ethanol, and ligated to a short oligonucleotide with the sequence GAATTCTAATACGACTCACTATA (SEQ ID NO. 6) using a splint-ligation method, T4 DNA ligase (Jena Bioscience), and a splint oligonucleotide with the sequence GTCGCCATCTCTTCCTATAGTGAGTCGTATTAG (SEQ ID NO. 7). All oligonucleotides were kept at a concentration of 2.5 µM. Molecules were then separated by gel shift using 6% Urea-PAGE, and molecules of the size 120 nt (corresponding to ligated pool members) were excised from the gel, eluted, and precipitated with ethanol. These molecules were then amplified by PCR using Taq Polymerase (Jena Bioscience) and the FWD1 (GAATTCTAATACGACTCACTATA, SEQ ID NO. 6) and REV1 (CCATCAGGATCAGCT, SEQ ID NO. 5) primers. The FWD1 primer contained a single RNA linkage at its 3' end so the reaction site of the deoxyribozyme could be regenerated. After another ethanol precipitation, the sense strands of the double-stranded molecules were base hydrolyzed, and the two strands were separated using 6% Urea-PAGE to regenerate the single-stranded DNA pool.

### Re-selection

A library was generated by randomly mutagenizing the most active deoxyribozyme isolated in the original selection:
GGAAGAGATGGCGACGACACAGGGACGATGCCGAATATCCTCAGTGCGCAGGGCCGCAG GGGGGAGTGACTTGGGATGGGGGGTCAGCTGATCCTGATGG (SEQ ID NO. 8) at a rate of 21% per position. The sequence of this library was **GGAAGAGATGGCGACGACACAGGGACGATGCCGAATATCCTCAGTGCGCAG GGCCGCAGGGGGGAGTGACTTGGGATGGGGGGTC**CACTAATGATCTGCCCGATG (SEQ ID NO. 9) (mutagenized positions are indicated in bold). This library was ordered from IDT and purified by PAGE. 10¹⁴ different sequences from this pool were mixed with the blocking oligonucleotide BO2 (CATCGGGCAGATCATTAGTG, SEQ ID NO. 10) in water, heated at 65 °C for 2 minutes and cooled at RT for 5 minutes. The mixture was then incubated for 10 minutes (round 1 - 3) or 1 minute (round 4-7) with CDP-Star at the following final concentrations: 1 µM DNA pool, 1.5 µM blocking oligonucleotide (BO2), 1× selection buffer, and 1 mM CDP-Star. DNA was then ethanol precipitated, and ligated to a short oligonucleotide with the sequence ACCGCTCAGGTGTAGTATCA (SEQ ID NO. 11) using a splint-ligation method, T4 DNA ligase (Jena Bioscience GmbH), and a splint oligonucleotide with the sequence GTCGCCATCTCTTCCTGATACTACACCTGAGCGGT (SEQ ID NO. 12). All oligonucleotides were kept at a concentration of 2.5 µM. Molecules were then separated by gel shift using 6% Urea-PAGE, and molecules of the size of 125 nt (corresponding to ligated pool members) were excised from the gel, eluted, and precipitated with ethanol. These molecules were then amplified by PCR using Q5 Hot Start Polymerase (NEB) and the FWD2 (ACCGCTCAGGTGTAGTATCA, SEQ ID NO. 13) and REV2 (CATCGGGCAGATCATTAGTG, SEQ ID NO. 10) primers. The FWD2 primer contained a single RNA linkage at its 3' end so the reaction site of the deoxyribozyme could be regenerated, and the REV2 primer contained a 5' phosphate group so the reverse strand could be selectively degraded. The reactions were then purified using the NucleoSpin Gel and PCR Clean-up kit (Macharey-Nagel), and the reverse strand was removed by digestion with λ-exonuclease (NEB). After a second purification with the NucleoSpin Gel and PCR Clean-up kit, the remaining sense strands were base hydrolyzed, and purified using 6% Urea-PAGE to regenerate single-stranded DNA pool.

### Example 2: Consensus sequence based on an alignment of 135,000 light-producing deoxyribozyme variants isolated by in vitro selection.

After 6 rounds of the reselection the evolved pool was characterized by high-throughput sequencing. The analysis was performed using the Illumina HiSeq System (2×150 bp, paired-end; NGSelect Amplicon product by Eurofins Genomics). Raw reads were processed with the cutadapt tool to remove adapter and primer sequences, perform quality trimming, and filter low quality reads. Paired-end reads were oriented, merged with the program fastq-join, and aligned using Clustal Omega. Data quality was evaluated using the FastQC tool. The secondary structure of the deoxyribozyme was predicted using comparative sequence analysis, which included frequency calculations of all di- and trinucleotide combinations and mutual information analysis (Gutell,R.R., Power,A., Hertz,G.Z., Putz,E.J. and Stormo,G.D. (1992). Nuc Acids Res, 20, 5785-5795) using in-house scripts. Analysis of conservation led to the identification of the consensus sequence 5' GGAAGA-(26 variable nucleotides)-GAATATCCCC-(18 variable nucleotides)-GGGGAGTGACTTGGGATGGGGG 3' (SEQ ID NO. 14). Detailed information about all tools used is listed in Table 1.

**Table 1. Tools used for data analysis**

| **Tool** | **Version** | **Source** |
|---|---|---|
| cutadapt | 1.15 | https://cutadapt.readthedocs.io/en/stable/ |
| fastq-join | 1.3.1 | https://github.com/brwnj/fastq join |
| Clustal Omega | 1.2.4 | https://www.ebi.ac.uk/Tools/msa/clustalo/ |
| FastQC | 0.11.5 | https://www.bioinformatics.babraham.ac.uk/projects/fastqc/ |
| ViennaRNA | 2.4.12 | https://www.tbi.univie.ac.at/RNA/ |

### Example 3: Measuring deoxyribozyme catalytic activity using a ligation assay

Oligonucleotides were ordered from Sigma-Aldrich and purified by PAGE or HPLC prior to use. Each deoxyribozyme was mixed with either water alone or water and the appropriate blocking oligonucleotide (indicated in Examples 5 and 6), heated at 65 °C for 2 minutes, and cooled at RT for 10 minutes. After that, 5× Selection Buffer and CDP-Star^{™} were added. Final concentrations were 1 µM deoxyribozyme, 1.5 µM blocking oligonucleotide (when needed), 1× Selection Buffer (50 mM HEPES pH 7.4, 200 mM KCl, 1 mM ZnCl₂, 1 µM Ce(SO₄)₂, 0.1 µM PbCl₂), and 1 mM CDP-Star^{™}. Reactions were incubated for various times in the dark at RT and ethanol-precipitated. Deoxyribozymes were then ligated to a short oligonucleotide as described in Example 1. Reacted and unreacted molecules were then separated on 6% PAGE gels, and the amount of ligated product was determined using ImageQuant TL software (GE Healthcare Life Sciences).

### Example 4: Generation of a chemiluminescent signal using the deoxyribozyme

Sequences of the isolated deoxyribozymes were ordered from Sigma-Aldrich and purified by PAGE or HPLC prior to use. Deoxyribozymes were mixed with either water or water and the appropriate blocking oligonucleotide, heated at 65 °C for 2 minutes, and cooled at RT for 10 minutes. After that, 5× Selection Buffer was added, and samples were transferred to a white half-area 96-well plate (Corning). CDP-Star^{™} was added, and chemiluminescence was measured for 1 to 24 hours using a Tecan Spark plate reader (Tecan Group). Final concentrations were 1 µM deoxyribozyme, 1.5 µM blocking oligonucleotide (when necessary), 1× Selection Buffer (50 mM HEPES pH 7.4, 200 mM KCl, 1 mM ZnCl₂, 1 µM Ce(SO₄)₂, 0.1 µM PbCl₂), and 1 mM CDP-Star^{™}.

### Example 5: Catalytic activity of deoxyribozymes from the initial selection

Deoxyribozymes from the initial selection were tested as described in Example 4, except using 0.25 mM rather than 1 mM CDP-Star^{™} substrate. The most active variant was 
GGAAGAGATGGCGACGACACAGGGACGATGCCGAATATCCTCAGTGCGCAGGGCCGCAG
GGGGGAGTGACTTGGGATGGGGGGTCAGCTGATCCTGATGG (SEQ ID NO. 8),
 and it generated light with a rate enhancement of 39.6-fold relative to the reaction in the absence of deoxyribozyme. The blocking oligonucleotide used was REV1 (CCATCAGGATCAGCT, SEQ ID NO. 5).

### Example 6: Catalytic activity of deoxyribozymes from the reselection.

The ten most abundant deoxyribozymes in the reselection were tested as described in Example 4, except using 0.25 mM rather than 1 mM CDP-Star^{™} substrate. The most abundant deoxyribozyme was 
GGAAGATATGGCGCGAACAAATGGACAATGCCGAATATCCCCCGCACGCAGGGCAACAA
GGGGGAGTGACTTGGGATGGGGGCTGCACTAATGATCTGCCCGATG (SEQ ID NO. 15)
 and it generated light with a rate enhancement of 97.9-fold relative to the reaction in the absence of deoxyribozyme. The blocking oligo used was REV2 (CATCGGGCAGATCATTAGTG, SEQ ID NO. 10).

The second most abundant deoxyribozyme was 
GGAAGAAATGACGAGGCCACACGGACGAGGCTGAATATCCTCACTGCGGTGTGCCCCAG
GGAGGAGTGACTTGGGATGGTGGGTTCACTAATGATCTGCCCGATG (SEQ ID NO. 16)
 and it generated light with a rate enhancement of 130.4-fold relative to the reaction in the absence of deoxyribozyme. The blocking oligo used was REV2 (CATCGGGCAGATCATTAGTG, SEQ ID NO. 10).

The third most abundant deoxyribozyme was 
GGAAGATAGTGTGTCGATAAAGGGACAATGCTGAATTTCCTCCCTGAGCCGGCGCGTAG
GGGGGAGTGACTTGGGATGGGGGGTGCACTAATGATCTGCCCGATG (SEQ ID NO. 17)
 and it generated light with a rate enhancement of 187-fold relative to the reaction in the absence of deoxyribozyme. The blocking oligo used was REV2 (CATCGGGCAGATCATTAGTG, SEQ ID NO. 10).

The fourth abundant deoxyribozyme was 
GGAAGACTTGACGAGCGCACAGCGCTTGTTCAGAATATCACCCGTCATGTGTTCGTGAAG
GGGGAGTGACTTGGGATGGGGGTTCCACTAATGATCTGCCCGATG (SEQ ID NO. 18)
 and it generated light with a rate enhancement of 129.2-fold relative to the reaction in the absence of deoxyribozyme. The blocking oligo used was REV2 (CATCGGGCAGATCATTAGTG, SEQ ID NO. 10).

The fifth most abundant deoxyribozyme was 
GGAAGAAATGGTGCGACACAGGGGCGCTCCAGAATATCCTCATTGCGATATGCCGCAGG
GAGGAGTGACTTGGGATGGTGGGTCCACTAATGATCTGCCCGATG (SEQ ID NO. 19)
 and it generated light with a rate enhancement of 169.3-fold relative to the reaction in the absence of deoxyribozyme. The blocking oligo used was REV2 (CATCGGGCAGATCATTAGTG, SEQ ID NO. 10).

The sixth most abundant deoxyribozyme was 
GGAAGAAATGTAGAGGAAACAGTGACTCTGCAGAATATCCTCACTGCGTAGTGGGGCAG
GGGGGAGTGACTTGGGATGGGGGCTACACTAATGATCTGCCCGATG (SEQ ID NO. 20)
 and it generated light with a rate enhancement of 186.5-fold relative to the reaction in the absence of deoxyribozyme. The blocking oligo used was REV2 (CATCGGGCAGATCATTAGTG, SEQ ID NO. 10).

The seventh most abundant deoxyribozyme was 
GGAAGAAACGCGACGACACAGTAATGATGCGGAATATCCCCCCTTCATAGGGCGAAAGG
GGGGAGTGACTTGGGATGGGGGTTACACTAATGATCTGCCCGATG (SEQ ID NO. 21)
 and it generated light with a rate enhancement of 116.7-fold relative to the reaction in the absence of deoxyribozyme. The blocking oligo used was REV2 (CATCGGGCAGATCATTAGTG, SEQ ID NO. 10).

The eighth most abundant deoxyribozyme was 
GGCAGAGATGGCAACGTCAACATGAGGATGCCGCATATCCCCAGTGCACACTTGGGCAG
TGGGGAGTGACTTGGGATGGGGGAAACACTAATGATCTGCCCGATG (SEQ ID NO. 22)
 and it generated light with a rate enhancement of 155.8-fold relative to the reaction in the absence of deoxyribozyme. The blocking oligo used was REV2 (CATCGGGCAGATCATTAGTG, SEQ ID NO. 10).

The ninth most abundant deoxyribozyme was 
GGAAGATATGGCGGTGACTCTTCGAAGCTGCCGAATATCCCCCGTGCGCGGAGCCTCAGG
GGGGAGTGACTTGGGATGGGGGGTCCACTAATGATCTGCCCGATG (SEQ ID NO. 23)
 and it generated light with a rate enhancement of 135.5-fold relative to the reaction in the absence of deoxyribozyme. The blocking oligo used was REV2 (CATCGGGCAGATCATTAGTG, SEQ ID NO. 10).

The tenth most abundant deoxyribozyme was 
GGAAGAAATGCCGACCTCCCAGGGTGGAGGCTGAATATCCACCATGGTCCGGAACGGAT
GGGGGAGTGACTTGGGATGGGGGAACCACTAATGATCTGCCCGATG (SEQ ID NO. 24)
 and it generated light with a rate enhancement of 152.6-fold relative to the reaction in the absence of deoxyribozyme. The blocking oligo used was REV2 (CATCGGGCAGATCATTAGTG, SEQ ID NO. 10).

### Example 7: Identification of the catalytic core of the deoxyribozyme.

To identify the catalytic core of the deoxyribozyme, variable regions in the sequence alignment of deoxyribozymes from the reselection (Example 1) were either deleted or replaced with AAAA linkers. The minimized deoxyribozyme also contained a T to C mutation at position 15 in helix 1 (numbering corresponds to that used in Figure 4) which occurred frequently in deoxyribozymes from the reselection. This deoxyribozyme was tested as described in Examples 3 and 4, except using 0.25 mM rather than 1 mM CDP-Star^{™} substrate.

The sequence of the minimized catalytic core of the deoxyribozyme is GGAAGAAAAAGAATATCCCCAAAAGGGGAGTGACTTGGGATGGGGG (SEQ ID NO. 25) The amount of product in the ligation assay was 34.6% relative to a control oligonucleotide containing a 5' phosphate. It also generated 128-fold more light than the background reaction in the absence of deoxyribozyme in the light-production assay. The catalytic activities of the variants described in Examples 8-13 are expressed relative to the activity of this construct.

### Example 8: Confirming the secondary structure model of the deoxyribozyme.

To confirm the triple helix in the secondary structure model of the deoxyribozyme, a series of mutants were constructed in which a proposed base triple involving positions 15, 18, and 36 (numbering corresponds to that used in Figure 4) was disrupted in various ways in single and double mutants, and restored in a triple mutant made up of mutations that by themselves disrupt the triple. Deoxyribozymes were tested as described in Example 4, except using 0.25 mM rather than 1 mM CDP-Star^{™} substrate.

The first mutant in the series GGAAGAAAAAGAATATCCCCAAAAGGGGAGTGACTTGGGATGGGGG (SEQ ID NO. 25) contains a canonical triple (15C 18G 36G), and it generated 100% as much light as the most common minimized catalytic core in the reselection (SEQ ID NO. 25, which is the same sequence).

The second mutant in the series GGAAGAAAAAGAATATCCTCAAAAGGGGAGTGACTTGGGATGGGGG (SEQ ID NO. 26) contains a disrupted triple (15T 18G 36G), and it generated 33.6% as much light as the most common minimized catalytic core in the reselection (SEQ ID NO. 25).

The third mutant in the series GGAAGAAAAAGAATATCCCCAAAAGAGGAGTGACTTGGGATGGGGG (SEQ ID NO. 27) contains a disrupted triple (15C 18A 36G), and it generated 4.9% as much light as the most common minimized catalytic core in the reselection (SEQ ID NO. 25).

The fourth mutant in the series GGAAGAAAAAGAATATCCCCAAAAGGGGAGTGACTTGGGATGGTGG (SEQ ID NO. 28) contains a disrupted triple (15C 18G 36T), and it generated 28.3% as much light as the most common minimized catalytic core in the reselection (SEQ ID NO. 25).

The fifth mutant in the series GGAAGAAAAAGAATATCCTCAAAAGAGGAGTGACTTGGGATGGGGG (SEQ ID NO. 29) contains a disrupted triple (15T 18A 36G), and it generated 11.8% as much light as the most common minimized catalytic core in the reselection (SEQ ID NO. 25).

The sixth mutant in the series
SEQ ID NO:20 = GGAAGAAAAAGAATATCCTCAAAAGGGGAGTGACTTGGGATGGTGG (SEQ ID NO. 30) contains a disrupted triple (15T 18G 36T), and it generated 7.1% as much light as the most common minimized catalytic core in the reselection (SEQ ID NO. 25).

The seventh mutant in the series GGAAGAAAAAGAATATCCCCAAAAGAGGAGTGACTTGGGATGGTGG (SEQ ID NO. 31) contains a disrupted triple (15C 18A 36T), and it generated 7.7% as much light as the most common minimized catalytic core in the reselection (SEQ ID NO. 25).

The eighth mutant in the series GGAAGAAAAAGAATATCCTCAAAAGAGGAGTGACTTGGGATGGTGG (SEQ ID NO. 32) contains a canonical triple (15T 18A 36T), and it generated 72.9% as much light as the most common minimized catalytic core in the reselection (SEQ ID NO. 25).

### Example 9: Catalytic activity of minimized catalytic cores containing point mutations in unpaired regions which occurred frequently in deoxyribozymes from the reselection.

Minimized catalytic cores containing point mutations in unpaired regions (positions 1-11, 22-33, and 38; numbering as in Figure 4) which occurred frequently in deoxyribozymes from the reselection were tested as described in Example 4, except using 0.25 mM rather than 1 mM CDP-Star^{™} substrate. These catalytic cores correspond to sequences in which R¹ was replaced by AAAA, R² was replaced by AAAA, and R³ was deleted. The results are summarized in the text as well as in Table 2 below.

**Table 2. Preferred nucleotides at unpaired positions in the deoxyribozyme. Enrichment is the frequency of the mutation in the evolved library divided by its frequency in the starting library. Only mutations with enrichment values ≥ 3.1×10⁻³ are shown. Percent activity is the amount of light produced by a variant of SEQ ID NO. 25 containing the indicated mutation relative to SEQ ID NO. 25 (which is normalized to a value of 100%).**

| **Position** | **Nucleotide** | **Enrichment** | **% activity** |
|---|---|---|---|
| 1 | G | 1.2 | 100 |
| | T | 4.7×10⁻³ | 9.9 |
| 2 | G | 1.2 | 100 |
| | T | 3.1×10⁻³ | 11 |
| 3 | A | 1.2 | 100 |
| | C | 1.1 | 106.2 |
| 4 | A | 1.3 | 100 |
| 5 | G | 1.3 | 100 |
| | T | 3.6×10⁻³ | 14 |
| 6 | A | 1.3 | 100 |
| | T | 3×10⁻¹ | 77 |
| 7 | G | 1.7 | 100 |
| | T | 2.7×10⁻² | 13.1 |
| | A | 3.3×10⁻³ | 18.5 |
| 8 | A | 1.3 | 100 |
| | C | 1 | 5.3 |
| | G | 1.4×10⁻¹ | 13.2 |
| | T | 1.1×10⁻¹ | 25 |
| 9 | A | 1.5 | 100 |
| | T | 1.4×10⁻¹ | 21.4 |
| | G | 1.8×10⁻² | 21.9 |
| 10 | T | 1.4 | 100 |
| | A | 1.4×10⁻¹ | 54.7 |
| | C | 5.6×10⁻² | 81.5 |
| | G | 1.7×10⁻² | 32.7 |
| 11 | A | 1.4 | 100 |
| | T | 5×10⁻¹ | 42.8 |
| | G | 1.6×10⁻¹ | 34.3 |
| 22 | G | 1.7 | 100 |
| | A | 8.5×10⁻³ | 43 |
| 23 | T | 1.5 | 100 |
| 24 | G | 1.7 | 100 |
| 25 | A | 1.5 | 100 |
| 26 | C | 1.5 | 100 |
| 27 | T | 1.5 | 100 |
| | C | 1.3×10⁻² | 76 |
| | G | 4.7×10⁻³ | 14.1 |
| 28 | T | 1.5 | 100 |
| 29 | G | 1.7 | 100 |
| 30 | G | 1.7 | 100 |
| 31 | G | 1.8 | 100 |
| 32 | A | 1.5 | 100 |
| 33 | T | 1.5 | 100 |
| | G | 6.3×10⁻³ | 20.5 |
| | C | 5.9×10⁻³ | 29.7 |
| 38 | G | 1.6 | 100 |
| | T | 3.3×10⁻¹ | 156.4 |
| | A | 1.4×10⁻¹ | 94.1 |
| | C | 5.3×10⁻² | 148.2 |

The first construct in this series contains the 1T mutation. It has the sequence TGAAGAAAAAGAATATCCCCAAAAGGGGAGTGACTTGGGATGGGGG (SEQ ID NO. 33) and it generated 9.9% as much light as the most common minimized catalytic core in the reselection (SEQ ID NO. 25).

The second construct in this series contains the 2T mutation. It has the sequence GTAAGAAAAAGAATATCCCCAAAAGGGGAGTGACTTGGGATGGGGG (SEQ ID NO. 34) and it generated 11% as much light as the most common minimized catalytic core in the reselection (SEQ ID NO. 25).

The third construct in this series contains the 3C mutation. It has the sequence GGCAGAAAAAGAATATCCCCAAAAGGGGAGTGACTTGGGATGGGGG (SEQ ID NO. 35) and it generated 106.2% as much light as the most common minimized catalytic core in the reselection (SEQ ID NO. 25).

The fourth construct in this series contains the 5T mutation. It has the sequence GGAATAAAAAGAATATCCCCAAAAGGGGAGTGACTTGGGATGGGGG (SEQ ID NO. 36) and it generated 14% as much light as the most common minimized catalytic core in the reselection (SEQ ID NO. 25).

The fifth construct in this series contains the 6T mutation. It has the sequence GGAAGTAAAAGAATATCCCCAAAAGGGGAGTGACTTGGGATGGGGG (SEQ ID NO. 37) and it generated 77% as much light as the most common minimized catalytic core in the reselection (SEQ ID NO. 25).

The sixth construct in this series contains the 7T mutation. It has the sequence GGAAGAAAAATAATATCCCCAAAAGGGGAGTGACTTGGGATGGGGG (SEQ ID NO. 38) and it generated 13.1% as much light as the most common minimized catalytic core in the reselection (SEQ ID NO. 25).

The seventh construct in this series contains the 7A mutation. It has the sequence GGAAGAAAAAAAATATCCCCAAAAGGGGAGTGACTTGGGATGGGGG (SEQ ID NO. 39) and it generated 18.5% as much light as the most common minimized catalytic core in the reselection (SEQ ID NO. 25).

The eighth construct in this series contains the 8C mutation. It has the sequence GGAAGAAAAAGCATATCCCCAAAAGGGGAGTGACTTGGGATGGGGG (SEQ ID NO. 40) and it generated 5.3% as much light as the most common minimized catalytic core in the reselection (SEQ ID NO. 25).

The ninth construct in this series contains the 8G mutation. It has the sequence GGAAGAAAAAGGATATCCCCAAAAGGGGAGTGACTTGGGATGGGGG (SEQ ID NO. 41) and it generated 13.2% as much light as the most common minimized catalytic core in the reselection (SEQ ID NO. 25).

The tenth construct in this series contains the 8T mutation. It has the sequence GGAAGAAAAAGTATATCCCCAAAAGGGGAGTGACTTGGGATGGGGG (SEQ ID NO. 42) and it generated 25% as much light as the most common minimized catalytic core in the reselection (SEQ ID NO. 25).

The eleventh construct in this series contains the 9T mutation. It has the sequence GGAAGAAAAAGATTATCCCCAAAAGGGGAGTGACTTGGGATGGGGG (SEQ ID NO. 43) and it generated 21.4% as much light as the most common minimized catalytic core in the reselection (SEQ ID NO. 25).

The twelfth construct in this series contains the 9G mutation. It has the sequence GGAAGAAAAAGAGTATCCCCAAAAGGGGAGTGACTTGGGATGGGGG (SEQ ID NO. 44) and it generated 21.9% as much light as the most common minimized catalytic core in the reselection (SEQ ID NO. 25).

The thirteenth construct in this series contains the 10A mutation. It has the sequence GGAAGAAAAAGAAAATCCCCAAAAGGGGAGTGACTTGGGATGGGGG (SEQ ID NO. 45) and it generated 54.7% as much light as the most common minimized catalytic core in the reselection (SEQ ID NO. 25).

The fourteenth construct in this series contains the 10C mutation. It has the sequence GGAAGAAAAAGAACATCCCCAAAAGGGGAGTGACTTGGGATGGGGG (SEQ ID NO. 46) and it generated 81.5% as much light as the most common minimized catalytic core in the reselection (SEQ ID NO. 25).

The fifteenth construct in this series contains the 10G mutation. It has the sequence GGAAGAAAAAGAAGATCCCCAAAAGGGGAGTGACTTGGGATGGGGG (SEQ ID NO. 47) and it generated 32.7% as much light as the most common minimized catalytic core in the reselection (SEQ ID NO. 25).

The sixteenth construct in this series contains the 11T mutation. It has the sequence GGAAGAAAAAGAATTTCCCCAAAAGGGGAGTGACTTGGGATGGGGG (SEQ ID NO. 48) and it generated 42.8% as much light as the most common minimized catalytic core in the reselection (SEQ ID NO. 25).

The seventeenth construct in this series contains the 11G mutation. It has the sequence GGAAGAAAAAGAATGTCCCCAAAAGGGGAGTGACTTGGGATGGGGG (SEQ ID NO. 49) and it generated 34.3% as much light as the most common minimized catalytic core in the reselection (SEQ ID NO. 25).

The eighteenth construct in this series contains the 22A mutation. It has the sequence GGAAGAAAAAGAATATCCCCAAAAGGGGAATGACTTGGGATGGGGG (SEQ ID NO. 50) and it generated 43% as much light as the most common minimized catalytic core in the reselection (SEQ ID NO. 25).

The nineteenth construct in this series contains the 27C mutation. It has the sequence GGAAGAAAAAGAATATCCCCAAAAGGGGAGTGACCTGGGATGGGGG (SEQ ID NO. 51) and it generated 76% as much light as the most common minimized catalytic core in the reselection (SEQ ID NO. 25).

The twentieth construct in this series contains the 27G mutation. It has the sequence GGAAGAAAAAGAATATCCCCAAAAGGGGAGTGACGTGGGATGGGGG (SEQ ID NO. 52) and it generated 14.1% as much light as the most common minimized catalytic core in the reselection (SEQ ID NO. 25).

The twenty first construct in this series contains the 33G mutation. It has the sequence GGAAGAAAAAGAATATCCCCAAAAGGGGAGTGACTTGGGAGGGGGG (SEQ ID NO. 53) and it generated 20.5% as much light as the most common minimized catalytic core in the reselection (SEQ ID NO. 25).

The twenty second construct in this series contains the 33C mutation. It has the sequence GGAAGAAAAAGAATATCCCCAAAAGGGGAGTGACTTGGGACGGGGG (SEQ ID NO. 54) and it generated 29.7% as much light as the most common minimized catalytic core in the reselection (SEQ ID NO. 25).

The twenty third construct in this series contains the 38T mutation. It has the sequence GGAAGAAAAAGAATATCCCCAAAAGGGGAGTGACTTGGGATGGGGT (SEQ ID NO. 55) and it generated 156.4% as much light as the most common minimized catalytic core in the reselection (SEQ ID NO. 25).

The twenty fourth construct in this series contains the 38A mutation. It has the sequence GGAAGAAAAAGAATATCCCCAAAAGGGGAGTGACTTGGGATGGGGA (SEQ ID NO. 56) and it generated 94.1% as much light as the most common minimized catalytic core in the reselection (SEQ ID NO. 25).

The twenty fifth construct in this series contains the 38C mutation. It has the sequence GGAAGAAAAAGAATATCCCCAAAAGGGGAGTGACTTGGGATGGGGC (SEQ ID NO. 57) and it generated 148.2% as much light as the most common minimized catalytic core in the reselection (SEQ ID NO. 25).

### Example 10: Catalytic activity of minimized catalytic cores containing different combinations of canonical base pairs and base triples.

Minimized catalytic cores containing different combinations of canonical base pairs (at the 12-21 base pair) and base triples (at the 13-20-34, 14-19-35, 15-18-36, and 16-17-37 base triples; numbering as in Figure 4) were tested as described in Example 4, except using 0.25 mM rather than 1 mM CDP-Star^{™} substrate. These catalytic cores correspond to sequences in which R¹ was replaced by AAAA, R² was replaced by AAAA, and R³ was deleted. The results are summarized in the text as well as in Table 3 below.

**Table 3. Activity of deoxyribozymes containing helices with different sequences and lengths. Percent activity is the amount of light produced by a variant of SEQ ID: 15 containing the indicated mutations relative to SEQ ID: 15 (which is normalized to a value of 100%).**

| **Positions** | **Pair or triple** | **% activity** |
|---|---|---|
| 12-21 | T-A | 100 |
| | A-T | 96 |
| | C-G | 12.2 |
| | G-C | 51.6 |
| 13-20-34 | All C-G:G | 100 |
| 14-19-35 | | |
| 15-18-36 | | |
| 16-17-37 | | |
| 13-20-34 | T-A:A | 15.1 |
| | T-A:T | 11 |
| 14-19-35 | T-A:A | 119.1 |
| | T-A:T | 45.4 |
| 15-18-36 | T-A:A | 61 |
| | T-A:T | 72.9 |
| 16-17-37 | T-A:A | 98.6 |
| | T-A:T | 141.7 |
| 14-19-35 | All T-A:A | 27.4 |
| 15-18-36 | | |
| 16-17-37 | | |
| 14-19-35 | All T-A:T | 34.9 |
| 15-18-36 | | |
| 16-17-37 | | |
| 13-20-34 | All T-A:A | 14.1 |
| 14-19-35 | | |
| 15-18-36 | | |
| 16-17-37 | | |
| 13-20-34 | All T-A:T | 12.5 |
| 14-19-35 | | |
| 15-18-36 | | |
| 16-17-37 | | |
| 16-17-37 | Deleted | 24.3 |
| 15-18-36 | Deleted | 3.3 |
| 16-17-37 | | |
| 14-19-35 | Deleted | 3.1 |
| 15-18-36 | | |
| 16-17-37 | | |

The first construct in this series contains T at position 12 and A at position 21. It has the sequence GGAAGAAAAAGAATATCCCCAAAAGGGGAGTGACTTGGGATGGGGG (SEQ ID NO. 25) and it generated 100% as much light as the most common minimized catalytic core in the reselection (SEQ ID NO. 25, which is the same sequence).

The second construct in this series contains A at position 12 and T at position 21. It has the sequence GGAAGAAAAAGAATAACCCCAAAAGGGGTGTGACTTGGGATGGGGG (SEQ ID NO. 58) and it generated 96% as much light as the most common minimized catalytic core in the reselection (SEQ ID NO. 25).

The third construct in this series contains C at position 12 and G at position 21. It has the sequence GGAAGAAAAAGAATACCCCCAAAAGGGGGGTGACTTGGGATGGGGG (SEQ ID NO. 59) and it generated 12.2% as much light as the most common minimized catalytic core in the reselection (SEQ ID NO. 25).

The fourth construct in this series contains G at position 12 and C at position 21. It has the sequence GGAAGAAAAAGAATAGCCCCAAAAGGGGCGTGACTTGGGATGGGGG (SEQ ID NO. 60) and it generated 51.6% as much light as the most common minimized catalytic core in the reselection (SEQ ID NO. 25).

The fifth construct in this series contains C-G:G base triples at positions 13-20-34, 14-19-35, 15-18-36, and 16-17-37. It has the sequence GGAAGAAAAAGAATATCCCCAAAAGGGGAGTGACTTGGGATGGGGG (SEQ ID NO. 25) and it generated 100% as much light as the most common minimized catalytic core in the reselection (SEQ ID NO. 25, which is the same sequence).

The sixth construct in this series contains a T-A:A base triple at position 13-20-34 and C-G:G triples at the other positions in the triple helix. It has the sequence GGAAGAAAAAGAATATTCCCAAAAGGGAAGTGACTTGGGATAGGGG (SEQ ID NO. 61) and it generated 15.1% as much light as the most common minimized catalytic core in the reselection (SEQ ID NO. 25).

The seventh construct in this series contains a T-A:T base triple at position 13-20-34 and C-G:G triples at the other positions in the triple helix. It has the sequence GGAAGAAAAAGAATATTCCCAAAAGGGAAGTGACTTGGGATTGGGG (SEQ ID NO. 62) and it generated 11% as much light as the most common minimized catalytic core in the reselection (SEQ ID NO. 25).

The eighth construct in this series contains a T-A:A base triple at position 14-19-35 and C-G:G triples at the other positions in the triple helix. It has the sequence GGAAGAAAAAGAATATCTCCAAAAGGAGAGTGACTTGGGATGAGGG (SEQ ID NO. 63) and it generated 119.1% as much light as the most common minimized catalytic core in the reselection (SEQ ID NO. 25).

The ninth construct in this series contains a T-A:T base triple at position 14-19-35 and C-G:G triples at the other positions in the triple helix. It has the sequence GGAAGAAAAAGAATATCTCCAAAAGGAGAGTGACTTGGGATGTGGG (SEQ ID NO. 64) and it generated 45.4% as much light as the most common minimized catalytic core in the reselection (SEQ ID NO. 25).

The tenth construct in this series contains a T-A:A base triple at position 15-18-36 and C-G:G triples at the other positions in the triple helix. It has the sequence GGAAGAAAAAGAATATCCTCAAAAGAGGAGTGACTTGGGATGGAGG (SEQ ID NO. 65) and it generated 61% as much light as the most common minimized catalytic core in the reselection (SEQ ID NO. 25).

The eleventh construct in this series contains a T-A:T base triple at position 15-18-36 and C-G:G triples at the other positions in the triple helix. It has the sequence GGAAGAAAAAGAATATCCTCAAAAGAGGAGTGACTTGGGATGGTGG (SEQ ID NO. 66) and it generated 72.9% as much light as the most common minimized catalytic core in the reselection (SEQ ID NO. 25).

The twelfth construct in this series contains a T-A:A base triple at position 16-17-37 and C-G:G triples at the other positions in the triple helix. It has the sequence GGAAGAAAAAGAATATCCCTAAAAAGGGAGTGACTTGGGATGGGAG (SEQ ID NO. 67) and it generated 98.6% as much light as the most common minimized catalytic core in the reselection (SEQ ID NO. 25).

The thirteenth construct in this series contains a T-A:T base triple at position 16-17-37 and C-G:G triples at the other positions in the triple helix. It has the sequence GGAAGAAAAAGAATATCCCTAAAAAGGGAGTGACTTGGGATGGGTG (SEQ ID NO. 68) and it generated 141.7% as much light as the most common minimized catalytic core in the reselection (SEQ ID NO. 25).

The fourteenth construct in this series contains a C-G:G base triple at position 13-20-34 and T-A:A base triples at position 14-19-35, 15-18-36, and 16-17-37. It has the sequence GGAAGAAAAAGAATATCTTTAAAAAAAGAGTGACTTGGGATGAAAG (SEQ ID NO. 69) and it generated 27.4% as much light as the most common minimized catalytic core in the reselection (SEQ ID NO. 25).

The fifteenth construct in this series contains a C-G:G base triple at position 13-20-34 and T-A:T base triples at position 14-19-35, 15-18-36, and 16-17-37. It has the sequence GGAAGAAAAAGAATATCTTTAAAAAAAGAGTGACTTGGGATGTTTG (SEQ ID NO. 70) and it generated 34.9% as much light as the most common minimized catalytic core in the reselection (SEQ ID NO. 25).

The sixteenth construct in this series contains T-A:A base triples at all positions in the triple helix. It has the sequence GGAAGAAAAAGAATATTTTTAAAAAAAAAGTGACTTGGGATAAAAG (SEQ ID NO. 71) and it generated 14.1% as much light as the most common minimized catalytic core in the reselection (SEQ ID NO. 25).

The seventeenth construct in this series contains T-A:T base triples at all positions in the triple helix. It has the sequence GGAAGAAAAAGAATATTTTTAAAAAAAAAGTGACTTGGGATTTTTG (SEQ ID NO. 72) and it generated 12.5% as much light as the most common minimized catalytic core in the reselection (SEQ ID NO. 25).

The eighteenth construct in this series contains a deleted triple at position 16-17-37 and C-G:G triples at the other positions in the triple helix. It has the sequence GGAAGAAAAAGAATATCCCAAAAGGGAGTGACTTGGGATGGGG (SEQ ID NO. 73) and it generated 24.3% as much light as the most common minimized catalytic core in the reselection (SEQ ID NO. 25).

The nineteenth construct in this series contains deleted triples at positions 16-17-37 and 15-18-36, and C-G:G triples at the other positions in the triple helix. It has the sequence GGAAGAAAAAGAATATCCAAAAGGAGTGACTTGGGATGGG (SEQ ID NO. 74) and it generated 3.3% as much light as the most common minimized catalytic core in the reselection (SEQ ID NO. 25).

The twentieth construct in this series contains deleted triples at positions 16-17-37, 15-18-36, and 14-19-35, and a C:G:G triple at position 13-20-34 in the triple helix. It has the sequence GGAAGAAAAAGAATATCAAAAGAGTGACTTGGGATGG (SEQ ID NO. 75) and it generated 3.1% as much light as the most common minimized catalytic core in the reselection (SEQ ID NO. 25).

### Example 11: Catalytic activity of minimized catalytic cores containing partially or completely disrupted base triples.

Minimized catalytic cores containing different partially or completely disrupted triples at the 13-20-34, 14-19-35, 15-18-36, and 16-17-37 base triples (numbering as in Figure 4) were tested as described in Example 4, except using 0.25 mM rather than 1 mM CDP-Star^{™} substrate. These catalytic cores correspond to sequences in which R¹ was replaced by AAAA, R² was replaced by AAAA, and R³ was deleted. The results are summarized in the text as well as in Table 4 below.

**Table 4. Activity of deoxyribozymes containing disrupted base triples. Percent activity is the amount of light produced by a variant of SEQ ID NO. 25 containing the indicated mutations relative to SEQ ID NO. 25 (which is normalized to a value of 100%). Asterisks indicate base triples with enrichment values (frequency in evolved library divided by frequency in starting library) ≥ 10⁻³.**

| **Sequence of triple** | **Type of triple** | **% activity** | | | |
|---|---|---|---|---|---|
| | | **13-20-34** | **14-19-35** | **15-18-36** | **16-17-37** |
| C-G:G | Canonical | 100* | 100* | 100* | 100* |
| T-A:T | | 11* | 45.4 | 72.9* | 141.7* |
| T-A:A | | 15.1 | 119.1* | 61* | 98.6* |
| A G:G | Partial Hoogsteen | 64* | 36* | 22.4* | 92.3 |
| G G:G | | 25 | 12.2 | 36 | 32.1 |
| T G:G | | 48.6 | 32 | 33.6* | 73.1* |
| A A:A | | 19.5 | 26.8 | 25.9 | 59.3 |
| C A:A | | 14.9 | 16.5 | 13.6 | 41.2 |
| G A:A | | 17.7 | 50.6 | 7.9* | 26.9 |
| A A:T | | 28.2 | 19.5 | 33.1 | 104.3 |
| C A:T | | 2.8 | 2.9 | 7.7* | 107.4 |
| G A:T | | 15 | 24.4 | 29 | 82.7 |
| C-G A | Partial Watson-Crick | 6.8 | 44.1 * | 18.4 | 50.9 |
| C-G T | | 6.4 | 54.2 | 28.3 | 71 |
| T-A G | | 3.8 | 3.5 | 11.8 | 29.6 |
| A-T A | Other | - | - | - | 89.1 |
| G-C A | | - | - | - | 38.7 |
| T G T | | 3.6 | 3.7 | 7.1 | 35.2 |
| G T A | | - | - | - | 22.7 |
| G G C | | 6.9 | 6.8 | 9.4* | 13.5 |
| C C G | | 6.8 | 6.5 | 5.8 | 7.2 |
| C A G | | 3.1 | 2.9 | 4.9 | 11.3 |

The first construct in the series contains an A G:G triple at position 13-20-34 in the triple helix. It has the sequence GGAAGAAAAAGAATATACCCAAAAGGGGAGTGACTTGGGATGGGGG (SEQ ID NO. 76) and it generated 64% as much light as the most common minimized catalytic core in the reselection (SEQ ID NO. 25).

The next construct in the series contains a G G:G triple at position 13-20-34 in the triple helix. It has the sequence GGAAGAAAAAGAATATGCCCAAAAGGGGAGTGACTTGGGATGGGGG (SEQ ID NO. 77) and it generated 25% as much light as the most common minimized catalytic core in the reselection (SEQ ID NO. 25).

The next construct in the series contains a T G:G triple at position 13-20-34 in the triple helix. It has the sequence GGAAGAAAAAGAATATTCCCAAAAGGGGAGTGACTTGGGATGGGGG (SEQ ID NO. 78) and it generated 48.6% as much light as the most common minimized catalytic core in the reselection (SEQ ID NO. 25).

The next construct in the series contains a A A:A triple at position 13-20-34 in the triple helix. It has the sequence GGAAGAAAAAGAATATACCCAAAAGGGAAGTGACTTGGGATAGGGG (SEQ ID NO. 79) and it generated 19.5% as much light as the most common minimized catalytic core in the reselection (SEQ ID NO. 25).

The next construct in the series contains a C A:A triple at position 13-20-34 in the triple helix. It has the sequence GGAAGAAAAAGAATATCCCCAAAAGGGAAGTGACTTGGGATAGGGG (SEQ ID NO. 80) and it generated 14.9% as much light as the most common minimized catalytic core in the reselection (SEQ ID NO. 25).

The next construct in the series contains a G A:A triple at position 13-20-34 in the triple helix. It has the sequence GGAAGAAAAAGAATATGCCCAAAAGGGAAGTGACTTGGGATAGGGG (SEQ ID NO. 81) and it generated 17.7% as much light as the most common minimized catalytic core in the reselection (SEQ ID NO. 25).

The next construct in the series contains a A A:T triple at position 13-20-34 in the triple helix. It has the sequence GGAAGAAAAAGAATATACCCAAAAGGGAAGTGACTTGGGATTGGGG (SEQ ID NO. 82) and it generated 28.2% as much light as the most common minimized catalytic core in the reselection (SEQ ID NO. 25).

The next construct in the series contains a C A:T triple at position 13-20-34 in the triple helix. It has the sequence GGAAGAAAAAGAATATCCCCAAAAGGGAAGTGACTTGGGATTGGGG (SEQ ID NO. 83) and it generated 2.8% as much light as the most common minimized catalytic core in the reselection (SEQ ID NO. 25).

The next construct in the series contains a G A:T triple at position 13-20-34 in the triple helix. It has the sequence GGAAGAAAAAGAATATGCCCAAAAGGGAAGTGACTTGGGATTGGGG (SEQ ID NO. 84) and it generated 15% as much light as the most common minimized catalytic core in the reselection (SEQ ID NO. 25).

The next construct in the series contains a C-G A triple at position 13-20-34 in the triple helix. It has the sequence GGAAGAAAAAGAATATCCCCAAAAGGGGAGTGACTTGGGATAGGGG (SEQ ID NO. 85) and it generated 6.8% as much light as the most common minimized catalytic core in the reselection (SEQ ID NO. 25).

The next construct in the series contains a C-G T triple at position 13-20-34 in the triple helix. It has the sequence GGAAGAAAAAGAATATCCCCAAAAGGGGAGTGACTTGGGATTGGGG (SEQ ID NO. 86) and it generated 6.4% as much light as the most common minimized catalytic core in the reselection (SEQ ID NO. 25).

The next construct in the series contains a T-A G triple at position 13-20-34 in the triple helix. It has the sequence GGAAGAAAAAGAATATTCCCAAAAGGGAAGTGACTTGGGATGGGGG (SEQ ID NO. 87) and it generated 3.8% as much light as the most common minimized catalytic core in the reselection (SEQ ID NO. 25).

The next construct in the series contains a T G T triple at position 13-20-34 in the triple helix. It has the sequence GGAAGAAAAAGAATATTCCCAAAAGGGGAGTGACTTGGGATTGGGG (SEQ ID NO. 88) and it generated 3.6% as much light as the most common minimized catalytic core in the reselection (SEQ ID NO. 25).

The next construct in the series contains a G G C triple at position 13-20-34 in the triple helix. It has the sequence GGAAGAAAAAGAATATGCCCAAAAGGGGAGTGACTTGGGATCGGGG (SEQ ID NO. 89) and it generated 6.9% as much light as the most common minimized catalytic core in the reselection (SEQ ID NO. 25).

The next construct in the series contains a C C G triple at position 13-20-34 in the triple helix. It has the sequence GGAAGAAAAAGAATATCCCCAAAAGGGCAGTGACTTGGGATGGGGG (SEQ ID NO. 90) and it generated 6.8% as much light as the most common minimized catalytic core in the reselection (SEQ ID NO. 25).

The next construct in the series contains a C A G triple at position 13-20-34 in the triple helix. It has the sequence GGAAGAAAAAGAATATCCCCAAAAGGGAAGTGACTTGGGATGGGGG (SEQ ID NO. 91) and it generated 3.1% as much light as the most common minimized catalytic core in the reselection (SEQ ID NO. 25).

The next construct in the series contains a A G:G triple at position 14-19-35 in the triple helix. It has the sequence GGAAGAAAAAGAATATCACCAAAAGGGGAGTGACTTGGGATGGGGG (SEQ ID NO. 92) and it generated 36% as much light as the most common minimized catalytic core in the reselection (SEQ ID NO. 25).

The next construct in the series contains a G G:G triple at position 14-19-35 in the triple helix. It has the sequence GGAAGAAAAAGAATATCGCCAAAAGGGGAGTGACTTGGGATGGGGG (SEQ ID NO. 93) and it generated 12.2% as much light as the most common minimized catalytic core in the reselection (SEQ ID NO. 25).

The next construct in the series contains a T G:G triple at position 14-19-35 in the triple helix. It has the sequence GGAAGAAAAAGAATATCTCCAAAAGGGGAGTGACTTGGGATGGGGG (SEQ ID NO. 94) and it generated 49% as much light as the most common minimized catalytic core in the reselection (SEQ ID NO. 25).

The next construct in the series contains a A A:A triple at position 14-19-35 in the triple helix. It has the sequence GGAAGAAAAAGAATATCACCAAAAGGAGAGTGACTTGGGATGAGGG (SEQ ID NO. 95) and it generated 26.8% as much light as the most common minimized catalytic core in the reselection (SEQ ID NO. 25).

The next construct in the series contains a C A:A triple at position 14-19-35 in the triple helix. It has the sequence GGAAGAAAAAGAATATCCCCAAAAGGAGAGTGACTTGGGATGAGGG (SEQ ID NO. 96) and it generated 16.5% as much light as the most common minimized catalytic core in the reselection (SEQ ID NO. 25).

The next construct in the series contains a G A:A triple at position 14-19-35 in the triple helix. It has the sequence GGAAGAAAAAGAATATCGCCAAAAGGAGAGTGACTTGGGATGAGGG (SEQ ID NO. 97) and it generated 50.6% as much light as the most common minimized catalytic core in the reselection (SEQ ID NO. 25).

The next construct in the series contains a A A:T triple at position 14-19-35 in the triple helix. It has the sequence GGAAGAAAAAGAATATCACCAAAAGGAGAGTGACTTGGGATGTGGG (SEQ ID NO. 98) and it generated 19.5% as much light as the most common minimized catalytic core in the reselection (SEQ ID NO. 25).

The next construct in the series contains a C A:T triple at position 14-19-35 in the triple helix. It has the sequence GGAAGAAAAAGAATATCCCCAAAAGGAGAGTGACTTGGGATGTGGG (SEQ ID NO. 99) and it generated 2.9% as much light as the most common minimized catalytic core in the reselection (SEQ ID NO. 25).

The next construct in the series contains a G A:T triple at position 14-19-35 in the triple helix. It has the sequence GGAAGAAAAAGAATATCGCCAAAAGGAGAGTGACTTGGGATGTGGG (SEQ ID NO. 100) and it generated 24.4% as much light as the most common minimized catalytic core in the reselection (SEQ ID NO. 25).

The next construct in the series contains a C-G A triple at position 14-19-35 in the triple helix. It has the sequence GGAAGAAAAAGAATATCCCCAAAAGGGGAGTGACTTGGGATGAGGG (SEQ ID NO. 101) and it generated 52.3% as much light as the most common minimized catalytic core in the reselection (SEQ ID NO. 25).

The next construct in the series contains a C-G T triple at position 14-19-35 in the triple helix. It has the sequence GGAAGAAAAAGAATATCCCCAAAAGGGGAGTGACTTGGGATGTGGG (SEQ ID NO. 102) and it generated 54.2% as much light as the most common minimized catalytic core in the reselection (SEQ ID NO. 25).

The next construct in the series contains a T-A G triple at position 14-19-35 in the triple helix. It has the sequence GGAAGAAAAAGAATATCTCCAAAAGGAGAGTGACTTGGGATGGGGG (SEQ ID NO. 103) and it generated 3.5% as much light as the most common minimized catalytic core in the reselection (SEQ ID NO. 25).

The next construct in the series contains a T G T triple at position 14-19-35 in the triple helix. It has the sequence GGAAGAAAAAGAATATCTCCAAAAGGGGAGTGACTTGGGATGTGGG (SEQ ID NO. 104) and it generated 3.7% as much light as the most common minimized catalytic core in the reselection (SEQ ID NO. 25).

The next construct in the series contains a G G C triple at position 14-19-35 in the triple helix. It has the sequence GGAAGAAAAAGAATATCGCCAAAAGGGGAGTGACTTGGGATGCGGG (SEQ ID NO. 105) and it generated 6.8% as much light as the most common minimized catalytic core in the reselection (SEQ ID NO. 25).

The next construct in the series contains a C C G triple at position 14-19-35 in the triple helix. It has the sequence GGAAGAAAAAGAATATCCCCAAAAGGCGAGTGACTTGGGATGGGGG (SEQ ID NO. 106) and it generated 6.5% as much light as the most common minimized catalytic core in the reselection (SEQ ID NO. 25).

The next construct in the series contains a C A G triple at position 14-19-35 in the triple helix. It has the sequence GGAAGAAAAAGAATATCCCCAAAAGGAGAGTGACTTGGGATGGGGG (SEQ ID NO. 107) and it generated 2.9% as much light as the most common minimized catalytic core in the reselection (SEQ ID NO. 25).

The next construct in the series contains a A G:G triple at position 15-18-36 in the triple helix. It has the sequence GGAAGAAAAAGAATATCCACAAAAGGGGAGTGACTTGGGATGGGGG (SEQ ID NO. 108) and it generated 22.4% as much light as the most common minimized catalytic core in the reselection (SEQ ID NO. 25).

The next construct in the series contains a G G:G triple at position 15-18-36 in the triple helix. It has the sequence GGAAGAAAAAGAATATCCGCAAAAGGGGAGTGACTTGGGATGGGGG (SEQ ID NO. 109) and it generated 36% as much light as the most common minimized catalytic core in the reselection (SEQ ID NO. 25).

The next construct in the series contains a T G:G triple at position 15-18-36 in the triple helix. It has the sequence GGAAGAAAAAGAATATCCTCAAAAGGGGAGTGACTTGGGATGGGGG (SEQ ID NO. 26) and it generated 33.6% as much light as the most common minimized catalytic core in the reselection (SEQ ID NO. 25).

The next construct in the series contains a A A:A triple at position 15-18-36 in the triple helix. It has the sequence GGAAGAAAAAGAATATCCACAAAAGAGGAGTGACTTGGGATGGAGG (SEQ ID NO. 110) and it generated 25.9% as much light as the most common minimized catalytic core in the reselection (SEQ ID NO. 25).

The next construct in the series contains a C A:A triple at position 15-18-36 in the triple helix. It has the sequence GGAAGAAAAAGAATATCCCCAAAAGAGGAGTGACTTGGGATGGAGG (SEQ ID NO. 111) and it generated 13.6% as much light as the most common minimized catalytic core in the reselection (SEQ ID NO. 25).

The next construct in the series contains a G A:A triple at position 15-18-36 in the triple helix. It has the sequence GGAAGAAAAAGAATATCCGCAAAAGAGGAGTGACTTGGGATGGAGG (SEQ ID NO. 112) and it generated 7.9% as much light as the most common minimized catalytic core in the reselection (SEQ ID NO. 25).

The next construct in the series contains a A A:T triple at position 15-18-36 in the triple helix. It has the sequence GGAAGAAAAAGAATATCCACAAAAGAGGAGTGACTTGGGATGGTGG (SEQ ID NO. 113) and it generated 33.1% as much light as the most common minimized catalytic core in the reselection (SEQ ID NO. 25).

The next construct in the series contains a C A:T triple at position 15-18-36 in the triple helix. It has the sequence GGAAGAAAAAGAATATCCCCAAAAGAGGAGTGACTTGGGATGGTGG (SEQ ID NO. 31) and it generated 7.7% as much light as the most common minimized catalytic core in the reselection (SEQ ID NO. 25).

The next construct in the series contains a G A:T triple at position 15-18-36 in the triple helix. It has the sequence GGAAGAAAAAGAATATCCGCAAAAGAGGAGTGACTTGGGATGGTGG (SEQ ID NO. 114) and it generated 29% as much light as the most common minimized catalytic core in the reselection (SEQ ID NO. 25).

The next construct in the series contains a C-G A triple at position 15-18-36 in the triple helix. It has the sequence GGAAGAAAAAGAATATCCCCAAAAGGGGAGTGACTTGGGATGGAGG (SEQ ID NO. 115) and it generated 18.4% as much light as the most common minimized catalytic core in the reselection (SEQ ID NO. 25).

The next construct in the series contains a C-G T triple at position 15-18-36 in the triple helix. It has the sequence GGAAGAAAAAGAATATCCCCAAAAGGGGAGTGACTTGGGATGGTGG (SEQ ID NO. 28) and it generated 28.3% as much light as the most common minimized catalytic core in the reselection (SEQ ID NO. 25).

The next construct in the series contains a T-A G triple at position 15-18-36 in the triple helix. It has the sequence GGAAGAAAAAGAATATCCTCAAAAGAGGAGTGACTTGGGATGGGGG (SEQ ID NO. 29) and it generated 11.8% as much light as the most common minimized catalytic core in the reselection (SEQ ID NO. 25).

The next construct in the series contains a T G T triple at position 15-18-36 in the triple helix. It has the sequence GGAAGAAAAAGAATATCCTCAAAAGGGGAGTGACTTGGGATGGTGG (SEQ ID NO. 30) and it generated 7.1% as much light as the most common minimized catalytic core in the reselection (SEQ ID NO. 25).

The next construct in the series contains a G G C triple at position 15-18-36 in the triple helix. It has the sequence GGAAGAAAAAGAATATCCGCAAAAGGGGAGTGACTTGGGATGGCGG (SEQ ID NO. 116) and it generated 9.4% as much light as the most common minimized catalytic core in the reselection (SEQ ID NO. 25).

The next construct in the series contains a C C G triple at position 15-18-36 in the triple helix. It has the sequence GGAAGAAAAAGAATATCCCCAAAAGCGGAGTGACTTGGGATGGGGG (SEQ ID NO. 117) and it generated 5.8% as much light as the most common minimized catalytic core in the reselection (SEQ ID NO. 25).

The next construct in the series contains a C A G triple at position 15-18-36 in the triple helix. It has the sequence GGAAGAAAAAGAATATCCCCAAAAGAGGAGTGACTTGGGATGGGGG (SEQ ID NO. 27) and it generated 4.9% as much light as the most common minimized catalytic core in the reselection (SEQ ID NO. 25).

The next construct in the series contains a A G:G triple at position 16-17-37 in the triple helix. It has the sequence GGAAGAAAAAGAATATCCCAAAAAGGGGAGTGACTTGGGATGGGGG (SEQ ID NO. 118) and it generated 92.3% as much light as the most common minimized catalytic core in the reselection (SEQ ID NO. 25).

The next construct in the series contains a G G:G triple at position 16-17-37 in the triple helix. It has the sequence GGAAGAAAAAGAATATCCCGAAAAGGGGAGTGACTTGGGATGGGGG (SEQ ID NO. 119) and it generated 32.1% as much light as the most common minimized catalytic core in the reselection (SEQ ID NO. 25).

The next construct in the series contains a T G:G triple at position 16-17-37 in the triple helix. It has the sequence GGAAGAAAAAGAATATCCCTAAAAGGGGAGTGACTTGGGATGGGGG (SEQ ID NO. 120) and it generated 73.1% as much light as the most common minimized catalytic core in the reselection (SEQ ID NO. 25).

The next construct in the series contains a A A:A triple at position 16-17-37 in the triple helix. It has the sequence GGAAGAAAAAGAATATCCCAAAAAAGGGAGTGACTTGGGATGGGAG (SEQ ID NO. 121) and it generated 59.3% as much light as the most common minimized catalytic core in the reselection (SEQ ID NO. 25).

The next construct in the series contains a C A:A triple at position 16-17-37 in the triple helix. It has the sequence GGAAGAAAAAGAATATCCCCAAAAAGGGAGTGACTTGGGATGGGAG (SEQ ID NO. 122) and it generated 41.2% as much light as the most common minimized catalytic core in the reselection (SEQ ID NO. 25).

The next construct in the series contains a G A:A triple at position 16-17-37 in the triple helix. It has the sequence GGAAGAAAAAGAATATCCCGAAAAAGGGAGTGACTTGGGATGGGAG (SEQ ID NO. 123) and it generated 26.9% as much light as the most common minimized catalytic core in the reselection (SEQ ID NO. 25).

The next construct in the series contains a A A:T triple at position 16-17-37 in the triple helix. It has the sequence GGAAGAAAAAGAATATCCCAAAAAAGGGAGTGACTTGGGATGGGTG (SEQ ID NO. 124) and it generated 104.3% as much light as the most common minimized catalytic core in the reselection (SEQ ID NO. 25).

The next construct in the series contains a C A:T triple at position 16-17-37 in the triple helix. It has the sequence GGAAGAAAAAGAATATCCCCAAAAAGGGAGTGACTTGGGATGGGTG (SEQ ID NO. 125) and it generated 107.4% as much light as the most common minimized catalytic core in the reselection (SEQ ID NO. 25).

The next construct in the series contains a G A:T triple at position 16-17-37 in the triple helix. It has the sequence GGAAGAAAAAGAATATCCCGAAAAAGGGAGTGACTTGGGATGGGTG (SEQ ID NO. 126) and it generated 82.7% as much light as the most common minimized catalytic core in the reselection (SEQ ID NO. 25).

The next construct in the series contains a C-G A triple at position 16-17-37 in the triple helix. It has the sequence GGAAGAAAAAGAATATCCCCAAAAGGGGAGTGACTTGGGATGGGAG (SEQ ID NO. 127) and it generated 50.9% as much light as the most common minimized catalytic core in the reselection (SEQ ID NO. 25).

The next construct in the series contains a C-G T triple at position 16-17-37 in the triple helix. It has the sequence GGAAGAAAAAGAATATCCCCAAAAGGGGAGTGACTTGGGATGGGTG (SEQ ID NO. 128) and it generated 71% as much light as the most common minimized catalytic core in the reselection (SEQ ID NO. 25).

The next construct in the series contains a T-A G triple at position 16-17-37 in the triple helix. It has the sequence GGAAGAAAAAGAATATCCCTAAAAAGGGAGTGACTTGGGATGGGGG (SEQ ID NO. 129) and it generated 29.6% as much light as the most common minimized catalytic core in the reselection (SEQ ID NO. 25).

The next construct in the series contains a A-T A triple at position 16-17-37 in the triple helix. It has the sequence GGAAGAAAAAGAATATCCCAAAAATGGGAGTGACTTGGGATGGGAG (SEQ ID NO. 130) and it generated 89.1% as much light as the most common minimized catalytic core in the reselection (SEQ ID NO. 25).

The next construct in the series contains a G-C A triple at position 16-17-37 in the triple helix. It has the sequence GGAAGAAAAAGAATATCCCGAAAACGGGAGTGACTTGGGATGGGAG (SEQ ID NO. 131) and it generated 38.7% as much light as the most common minimized catalytic core in the reselection (SEQ ID NO. 25).

The next construct in the series contains a T G T triple at position 16-17-37 in the triple helix. It has the sequence GGAAGAAAAAGAATATCCCTAAAAGGGGAGTGACTTGGGATGGGTG (SEQ ID NO. 132) and it generated 35.2% as much light as the most common minimized catalytic core in the reselection (SEQ ID NO. 25).

The next construct in the series contains a G T A triple at position 16-17-37 in the triple helix. It has the sequence GGAAGAAAAAGAATATCCCGAAAATGGGAGTGACTTGGGATGGGAG (SEQ ID NO. 133) and it generated 22.7% as much light as the most common minimized catalytic core in the reselection (SEQ ID NO. 25).

The next construct in the series contains a G G C triple at position 16-17-37 in the triple helix. It has the sequence GGAAGAAAAAGAATATCCCGAAAAGGGGAGTGACTTGGGATGGGCG (SEQ ID NO. 134) and it generated 13.5% as much light as the most common minimized catalytic core in the reselection (SEQ ID NO. 25).

The next construct in the series contains a C C G triple at position 16-17-37 in the triple helix. It has the sequence GGAAGAAAAAGAATATCCCCAAAACGGGAGTGACTTGGGATGGGGG (SEQ ID NO. 135) and it generated 7.2% as much light as the most common minimized catalytic core in the reselection (SEQ ID NO. 25).

The next construct in the series contains a C A G triple at position 16-17-37 in the triple helix. It has the sequence GGAAGAAAAAGAATATCCCCAAAAAGGGAGTGACTTGGGATGGGGG (SEQ ID NO. 136) and it generated 11.3% as much light as the most common minimized catalytic core in the reselection (SEQ ID NO. 25).

### Example 12: Catalytic activity of minimized catalytic cores of deoxyribozymes from the reselection.

The 50 most abundant minimized catalytic cores of deoxyribozymes in the reselection (cores 1-50) were tested as described in Example 4, except using 0.25 mM rather than 1 mM CDP-Star^{™} substrate. These correspond to sequences in which R¹ was replaced by AAAA, R² was replaced by AAAA, and R³ was deleted. Core 1 (SEQ ID NO. 25) is already described in Example 7, Cores 2 (SEQ ID NO:26) and 3 (SEQ ID NO:32) are already described in Example 8, Cores 8 (SEQ ID NO:40), 12 (SEQ ID NO:48), 19 (SEQ ID NO:55), 20 (SEQ ID NO:41), 21 (SEQ ID NO:42), 24 (SEQ ID NO:57), 39 (SEQ ID NO:47), 40 (SEQ ID NO:37), 42 (SEQ ID NO:46), and 48 (SEQ ID NO:56) are already described in Example 9, cores 16 (SEQ ID NO:66), 32 (SEQ ID NO:67), and 46 (SEQ ID NO:63) are already described in Example 10, and Cores 5 (SEQ ID NO:92), 6 (SEQ ID NO:108), 34 (SEQ ID NO:101), 41 (SEQ ID NO:94) are already described in Example 11. The remaining cores are listed here.

The sequence of core 4 is **GGAAGAAAAAGAATTTCCTCAAAAGGGGAGTGACTTGGGATGGGGG** (SEQ ID NO. 137) and it generated 5.6% as much light as the most common minimized catalytic core in the reselection (SEQ ID NO. 25).

The sequence of core 7 is GGCAGAAAAAGCATATCCCCAAAAGGGGAGTGACTTGGGATGGGGG (SEQ ID NO. 138) and it generated 8.2% as much light as the most common minimized catalytic core in the reselection (SEQ ID NO. 25).

The sequence of core 9 is GGAAGAAAAAGCATATCCTCAAAAGGGGAGTGACTTGGGATGGGGG (SEQ ID NO. 139) and it generated 4% as much light as the most common minimized catalytic core in the reselection (SEQ ID NO. 25).

The sequence of core 10 is GGAAGAAAAAGAATATCCGCAAAAGGGGAGTGACTTGGGATGGCGA (SEQ ID NO. 140) and it generated 4.4% as much light as the most common minimized catalytic core in the reselection (SEQ ID NO. 25).

The sequence of core 11 is GGAAGAAAAAGATAGTAACCAAAAGGGGAGTGACTTGGGATGGGGG (SEQ ID NO. 141) and it generated 2.5% as much light as the most common minimized catalytic core in the reselection (SEQ ID NO. 25).

The sequence of core 13 is GGAAGAAAAAGCATATCCCCAAAAGGGGAGTGACTTGGGATGGGGT (SEQ ID NO. 142) and it generated 6.2% as much light as the most common minimized catalytic core in the reselection (SEQ ID NO. 25).

The sequence of core 14 is GGAAGAAAAAGAATATCCTCAAAAGGGGAGTGACTTGGGATGGGGT (SEQ ID NO. 143) and it generated 51.2% as much light as the most common minimized catalytic core in the reselection (SEQ ID NO. 25).

The sequence of core 15 is GGAAGTAAAAGAATATCCTCAAAAGGGGAGTGACTTGGGATGGGGT (SEQ ID NO. 144) and it generated 36.1% as much light as the most common minimized catalytic core in the reselection (SEQ ID NO. 25).

The sequence of core 17 is GGAAGAAAAAGTATATCCTCAAAAGGGGAGTGACTTGGGATGGGGG (SEQ ID NO. 145) and it generated 14.4% as much light as the most common minimized catalytic core in the reselection (SEQ ID NO. 25).

The sequence of core 18 is GGAAGAAAAAGCATATCCACAAAAGGGGAGTGACTTGGGATGGGGG (SEQ ID NO. 146) and it generated 10.3% as much light as the most common minimized catalytic core in the reselection (SEQ ID NO. 25).

The sequence of core 22 is GGCAGTAAAAGCATATCCACAAAAGGGGAGTGACTTGGGATGGGGG (SEQ ID NO. 147) and it generated 9.5% as much light as the most common minimized catalytic core in the reselection (SEQ ID NO. 25).

The sequence of core 23 is GGAAGAAAAAGGATATCCTCAAAAGGGGAGTGACTTGGGATGGGGG (SEQ ID NO. 148) and it generated 14% as much light as the most common minimized catalytic core in the reselection (SEQ ID NO. 25).

The sequence of core 25 is GGAAGTAAAAGCATATCCTCAAAAGGGGAGTGACTTGGGATGGGGG (SEQ ID NO. 149) and it generated 10.5% as much light as the most common minimized catalytic core in the reselection (SEQ ID NO. 25).

The sequence of core 26 is GGAAGAAAAAGAATATCATCAAAAGGGGAGTGACTTGGGATGGGGG (SEQ ID NO. 150) and it generated 9.7% as much light as the most common minimized catalytic core in the reselection (SEQ ID NO. 25).

The sequence of core 27 is GGAAGAAAAATATCTACCCTAAAAGGGGAGTGACTTGGGATGGGGG (SEQ ID NO. 151) and it generated 8.9% as much light as the most common minimized catalytic core in the reselection (SEQ ID NO. 25).

The sequence of core 28 is GGAAGAAAAAGCATATCCCCAAAAGGGGAGTGACTTGGGATGAGGG (SEQ ID NO. 152) and it generated 12.7% as much light as the most common minimized catalytic core in the reselection (SEQ ID NO. 25).

The sequence of core 29 is GGAAGAAAAAGAATATCCTCAAAAGGGGAGTGACTTGGGATAGGGG (SEQ ID NO. 153) and it generated 21.5% as much light as the most common minimized catalytic core in the reselection (SEQ ID NO. 25).

The sequence of core 30 is GGAAGAAAAAGGATATCCACAAAAGGGGAGTGACTTGGGATGGGGG (SEQ ID NO. 154) and it generated 21.1% as much light as the most common minimized catalytic core in the reselection (SEQ ID NO. 25).

The sequence of core 31 is GGAAGAAAAAGCATATCCTCAAAAGAGGAGTGACTTGGGATGGTGG (SEQ ID NO. 155) and it generated 13.5% as much light as the most common minimized catalytic core in the reselection (SEQ ID NO. 25).

The sequence of core 33 is GGCAGAAAAAGAATATCCTCAAAAGGGGAGTGACTTGGGATGGGGG (SEQ ID NO. 156) and it generated 121.6% as much light as the most common minimized catalytic core in the reselection (SEQ ID NO. 25).

The sequence of core 35 is GGAAGTAAAAGAATATCACCAAAAGGGGAGTGACTTGGGATGGGGG (SEQ ID NO. 157) and it generated 50.2% as much light as the most common minimized catalytic core in the reselection (SEQ ID NO. 25).

The sequence of core 36 is GGAAGAAAAAGAACATCCTCAAAAGGGGAGTGACTTGGGATGGGGG (SEQ ID NO. 158) and it generated 27.4% as much light as the most common minimized catalytic core in the reselection (SEQ ID NO. 25).

The sequence of core 37 is GGAAGAAAAAGCATATACCCAAAAGGGGAGTGACTTGGGATGGGGG (SEQ ID NO. 159) and it generated 14.5% as much light as the most common minimized catalytic core in the reselection (SEQ ID NO. 25).

The sequence of core 38 is GGAAGAAAAAGCGTATACCCAAAAGGGGAGTGACTTGGGATGGGGC (SEQ ID NO. 160) and it generated 11.9% as much light as the most common minimized catalytic core in the reselection (SEQ ID NO. 25).

The sequence of core 43 is GGAAGTAAAAGAATTTCCCCAAAAGGGGAGTGACTTGGGATGGGGG (SEQ ID NO. 161) and it generated 71.5% as much light as the most common minimized catalytic core in the reselection (SEQ ID NO. 25).

The sequence of core 44 is GGAAGAAAAAGAATATCCTCAAAAGGGGAGTGACTTGGGATGGGGA (SEQ ID NO. 162) and it generated 44.5% as much light as the most common minimized catalytic core in the reselection (SEQ ID NO. 25).

The sequence of core 45 is GGAAGAAAAAGAATATCCTTAAAAGAGGAGTGACTTGGGATGGTGG (SEQ ID NO. 163) and it generated 60.4% as much light as the most common minimized catalytic core in the reselection (SEQ ID NO. 25).

The sequence of core 47 is GGCAGAAAAAGAATATCCCCAAAAGGGGAATGACTTGGGATGGGGG (SEQ ID NO. 164) and it generated 23.9% as much light as the most common minimized catalytic core in the reselection (SEQ ID NO. 25).

The sequence of core 49 is GGAAGAAAAAGAATATTCTCAAAAGGGGAGTGACTTGGGATGAGGG (SEQ ID NO. 165) and it generated 8.2% as much light as the most common minimized catalytic core in the reselection (SEQ ID NO. 25).

The sequence of core 50 is GGAAGAAAAAGAATTTCCCCAAAAGGGAAGTGACTTGGGATTGGGG (SEQ ID NO. 166) and it generated 8.5% as much light as the most common minimized catalytic core in the reselection (SEQ ID NO. 25).

### Example 13: Identification of sites in the deoxyribozyme at which new sequences can be inserted.

Deletion analysis (described in Example 7) showed that R¹ (originally 26 nucleotides) could be replaced by AAAA, that R² (originally 18 nucleotides) could be replaced by AAAA, and R³ (originally 18 nucleotides) could be deleted (see Figure 6 for the locations of R¹, R², and R³ mapped onto the secondary structure of the deoxyribozyme). To determine the extent to which other nucleotides could also be inserted at these positions, a series of constructs was generated in which random sequence regions of different lengths (N = 25% A, 25% C, 25% G, and 25% T) were inserted at R¹, R², or R³. Deoxyribozymes were tested as described in Example 4, except using 0.25 mM rather than 1 mM CDP-Star^{™} substrate. The notation N₃, N₁₀, N₃₀ means that the oligonucleotide tested contained a mix of sequences at the positions indicated with Ns - a particular oligonucleotide would have in each position a 25% chance of having an A, a 25% chance of having a C, a 25% chance of having G, and a 25% chance of having a T. This allows us to conclude that the sequence works regardless of the specific nucleotides in these positions.

The first deoxyribozyme in the series GGAAGAN₃GAATATCCCCAAAAGGGGAGTGACTTGGGATGGGGG (SEQ ID NO. 167) contained an N3 insertion at R¹. This generated 36.8% as much light as SEQ ID NO. 25 (a variant with an AAAA insertion at R¹).

The second deoxyribozyme in the series GGAAGAN₁₀GAATATCCCCAAAAGGGGAGTGACTTGGGATGGGGG (SEQ ID NO. 168) contained an N10 insertion at R¹. This generated 31.6% as much light as SEQ ID NO. 25 (a variant with an AAAA insertion at R¹).

The third deoxyribozyme in the series GGAAGAN₃₀GAATATCCCCAAAAGGGGAGTGACTTGGGATGGGGG (SEQ ID NO. 169) contained an N30 insertion at R¹. This generated 16.5% as much light as SEQ ID NO. 25 (a variant with an AAAA insertion at R¹).

The fourth deoxyribozyme in the series GGAAGAAAAAGAATATCCCCN₃GGGGAGTGACTTGGGATGGGGG (SEQ ID NO. 170) contained an N3 insertion at R². This generated 110.8% as much light as SEQ ID NO. 25 (a variant with an AAAA insertion at R²).

The fifth deoxyribozyme in the series GGAAGAAAAAGAATATCCCCN₁₀GGGGAGTGACTTGGGATGGGGG (SEQ ID NO. 171) contained an N10 insertion at R². This generated 68.4% as much light as SEQ ID NO. 25 (a variant with an AAAA insertion at R²).

The sixth deoxyribozyme in the series GGAAGAAAAAGAATATCCCCN₃₀GGGGAGTGACTTGGGATGGGGG (SEQ ID NO. 172) contained an N30 insertion at R². This generated 41.1% as much light as SEQ ID NO. 25 (a variant with an AAAA insertion at R²).

The seventh deoxyribozyme in the series GGAAGAAAAAGAATATCCCCAAAAGGGGAGTGACTTGGGATGGGGGN₃ (SEQ ID NO. 173) contained an N3 extension at at R³. This generated 84.5% as much light as SEQ ID NO. 25 (a variant with no extension at R³).

The eighth deoxyribozyme in the series GGAAGAAAAAGAATATCCCCAAAAGGGGAGTGACTTGGGATGGGGGN₁₀ (SEQ ID NO.174) contained an N10 extension at R³. This generated 52% as much light as SEQ ID NO. 25 (a variant with no extension at R³).

The ninth deoxyribozyme in the series GGAAGAAAAAGAATATCCCCAAAAGGGGAGTGACTTGGGATGGGGGN₃₀ (SEQ ID NO.175) contained an N30 extension at R³. This generated 29.1% as much light as SEQ ID NO. 25 (a variant with no extension at R³).

### Example 14: Further characterization of insertion sites in the deoxyribozyme.

To further characterize deoxyribozyme insertion sites, five different variants of each of the nine R¹-R²-R³ architectures described in Example 13 were generated in which arbitrary sequences were inserted at R¹, R², and/or R³. In addition, five different variants of each of two new R¹-R²-R³ architectures were generated. See Figure 6 for the locations of R¹, R², and R³ mapped onto the secondary structure of the deoxyribozyme. Deoxyribozymes were tested as described in Example 4, except using 0.25 mM rather than 1 mM CDP-Star^{™} substrate.

The first deoxyribozyme in the series GGAAGAAGAGAATATCCCCAAAAGGGGAGTGACTTGGGATGGGGG (SEQ ID NO. 176) contained an arbitrary 3 nucleotide sequence at R¹, AAAA at R², and no nucleotides at R³. This generated 108.2% as much light as SEQ ID NO. 25 (a variant with the sequence AAAA at R¹, AAAA at R², and no nucleotides at R³).

The next deoxyribozyme in the series GGAAGAACTGAATATCCCCAAAAGGGGAGTGACTTGGGATGGGGG (SEQ ID NO. 177) contained an arbitrary 3 nucleotide sequence at R¹, AAAA at R², and no nucleotides at R³. This generated 54.3% as much light as SEQ ID NO. 25.

The next deoxyribozyme in the series GGAAGATAAGAATATCCCCAAAAGGGGAGTGACTTGGGATGGGGG (SEQ ID NO. 178) contained an arbitrary 3 nucleotide sequence at R¹, AAAA at R², and no nucleotides at R³. This generated 169.1% as much light as SEQ ID NO. 25.

The next deoxyribozyme in the series GGAAGAACCGAATATCCCCAAAAGGGGAGTGACTTGGGATGGGGG (SEQ ID NO. 179) contained an arbitrary 3 nucleotide sequence at R¹, AAAA at R², and no nucleotides at R³. This generated 61.5% as much light as SEQ ID NO. 25.

The next deoxyribozyme in the series GGAAGACCAGAATATCCCCAAAAGGGGAGTGACTTGGGATGGGGG (SEQ ID NO. 180) contained an arbitrary 3 nucleotide sequence at R¹, AAAA at R², and no nucleotides at R³. This generated 70.8% as much light as SEQ ID NO. 25.

The next deoxyribozyme in the series GGAAGAGGGATGACCAGAATATCCCCAAAAGGGGAGTGACTTGGGATGGGGG (SEQ ID NO. 181)
contained an arbitrary 10 nucleotide sequence at R¹, AAAA at R², and no nucleotides at R³. This generated 29.6% as much light as SEQ ID NO. 25.

The next deoxyribozyme in the series GGAAGAACGTAATCGGGAATATCCCCAAAAGGGGAGTGACTTGGGATGGGGG (SEQ ID NO. 182)
contained an arbitrary 10 nucleotide sequence at R¹, AAAA at R², and no nucleotides at R³. This generated 49.1% as much light as SEQ ID NO. 25.

The next deoxyribozyme in the series GGAAGACCTCGCTACCGAATATCCCCAAAAGGGGAGTGACTTGGGATGGGGG (SEQ ID NO. 183)
contained an arbitrary 10 nucleotide sequence at R¹, AAAA at R², and no nucleotides at R³. This generated 25.3% as much light as SEQ ID NO. 25.

The next deoxyribozyme in the series GGAAGAGGGGGATCTTGAATATCCCCAAAAGGGGAGTGACTTGGGATGGGGG (SEQ ID NO. 184)
contained an arbitrary 10 nucleotide sequence at R¹, AAAA at R², and no nucleotides at R³. This generated 19.3% as much light as SEQ ID NO. 25.

The next deoxyribozyme in the series GGAAGATTAACGTCTCGAATATCCCCAAAAGGGGAGTGACTTGGGATGGGGG (SEQ ID NO. 185)
contained an arbitrary 10 nucleotide sequence at R¹, AAAA at R², and no nucleotides at R³. This generated 33.3% as much light as SEQ ID NO. 25.

The next deoxyribozyme in the series 
GGAAGACGCCCGCTGTTTTACAGTGCTGCAAAAAACGAATATCCCCAAAAGGGGAGTGA
CTTGGGATGGGGG (SEQ ID NO. 186)
 contained an arbitrary 30 nucleotide sequence at R¹, AAAA at R², and no nucleotides at R³. This generated 32.7% as much light as SEQ ID NO. 25.

The next deoxyribozyme in the series 
GGAAGAAGTCTGGCCGTCGAATCTGGCCTATGTATTGAATATCCCCAAAAGGGGAGTGA
CTTGGGATGGGGG (SEQ ID NO. 187)
 contained an arbitrary 30 nucleotide sequence at R¹, AAAA at R², and no nucleotides at R³. This generated 22.5% as much light as SEQ ID NO. 25.

The next deoxyribozyme in the series 
GGAAGACATAAGAGTGGTGGCGACCGTTACAATTAGGAATATCCCCAAAAGGGGAGTGA
CTTGGGATGGGGG (SEQ ID NO. 188)
 contained an arbitrary 30 nucleotide sequence at R¹, AAAA at R², and no nucleotides at R³. This generated 29.8% as much light as SEQ ID NO. 25.

The next deoxyribozyme in the series 
GGAAGAAATGGGCTCGAATTAACGGGTAGTTAATTAGAATATCCCCAAAAGGGGAGTGA
CTTGGGATGGGGG (SEQ ID NO. 189)
 contained an arbitrary 30 nucleotide sequence at R¹, AAAA at R², and no nucleotides at R³. This generated 15.7% as much light as SEQ ID NO. 25.

The next deoxyribozyme in the series 
GGAAGAAGGTGATAGGGGAGCGCACGGAATTACCCAGAATATCCCCAAAAGGGGAGTG
ACTTGGGATGGGGG (SEQ ID NO. 190)
 contained an arbitrary 30 nucleotide sequence at R¹, AAAA at R², and no nucleotides at R³. This generated 14.9% as much light as SEQ ID NO. 25.

The next deoxyribozyme in the series GGAAGAAAAAGAATATCCCCGGAGGGGAGTGACTTGGGATGGGGG (SEQ ID NO. 191) contained AAAA at R¹, an arbitrary 3 nucleotide sequence at R², and no nucleotides at R³. This generated 116.5% as much light as SEQ ID NO. 25.

The next deoxyribozyme in the series GGAAGAAAAAGAATATCCCCGAGGGGGAGTGACTTGGGATGGGGG (SEQ ID NO. 192) contained AAAA at R¹, an arbitrary 3 nucleotide sequence at R², and no nucleotides at R³. This generated 96.7% as much light as SEQ ID NO. 25.

The next deoxyribozyme in the series GGAAGAAAAAGAATATCCCCTAAGGGGAGTGACTTGGGATGGGGG (SEQ ID NO. 193) contained AAAA at R¹, an arbitrary 3 nucleotide sequence at R², and no nucleotides at R³. This generated 151.2% as much light as SEQ ID NO. 25.

The next deoxyribozyme in the series GGAAGAAAAAGAATATCCCCGCTGGGGAGTGACTTGGGATGGGGG (SEQ ID NO. 194) contained AAAA at R¹, an arbitrary 3 nucleotide sequence at R², and no nucleotides at R³. This generated 127.9% as much light as SEQ ID NO. 25.

The next deoxyribozyme in the series GGAAGAAAAAGAATATCCCCAATGGGGAGTGACTTGGGATGGGGG (SEQ ID NO. 195) contained AAAA at R¹, an arbitrary 3 nucleotide sequence at R², and no nucleotides at R³. This generated 101.9% as much light as SEQ ID NO. 25.

The next deoxyribozyme in the series GGAAGAAAAAGAATATCCCCGGGAAGGCCTGGGGAGTGACTTGGGATGGGGG (SEQ ID NO. 196)
contained AAAA at R¹, an arbitrary 10 nucleotide sequence at R², and no nucleotides at R³. This generated 104% as much light as SEQ ID NO. 25.

The next deoxyribozyme in the series GGAAGAAAAAGAATATCCCCTTTTACTGTGGGGGAGTGACTTGGGATGGGGG (SEQ ID NO. 197)
contained AAAA at R¹, an arbitrary 10 nucleotide sequence at R², and no nucleotides at R³. This generated 92.8% as much light as SEQ ID NO. 25.

The next deoxyribozyme in the series GGAAGAAAAAGAATATCCCCCTTAGCAAATGGGGAGTGACTTGGGATGGGGG (SEQ ID NO. 198)
contained AAAA at R¹, an arbitrary 10 nucleotide sequence at R², and no nucleotides at R³. This generated 59.9% as much light as SEQ ID NO. 25.

The next deoxyribozyme in the series GGAAGAAAAAGAATATCCCCAAAAAGCATGGGGGAGTGACTTGGGATGGGGG (SEQ ID NO. 199)
contained AAAA at R¹, an arbitrary 10 nucleotide sequence at R², and no nucleotides at R³. This generated 86% as much light as SEQ ID NO. 25.

The next deoxyribozyme in the series GGAAGAAAAAGAATATCCCCAATTTCACCCGGGGAGTGACTTGGGATGGGGG (SEQ ID NO. 200)
contained AAAA at R¹, an arbitrary 10 nucleotide sequence at R², and no nucleotides at R³. This generated 36.2% as much light as SEQ ID NO. 25.

The next deoxyribozyme in the series 
GGAAGAAAAAGAATATCCCCTAACGAGTCTCAAAAGCTACTTCGCTATAAGGGGAGTGA
CTTGGGATGGGGG (SEQ ID NO. 201)
 contained AAAA at R¹, an arbitrary 30 nucleotide sequence at R², and no nucleotides at R³. This generated 50.4% as much light as SEQ ID NO. 25.

The next deoxyribozyme in the series 
GGAAGAAAAAGAATATCCCCGGGTGACACCACCGATAACGAGCCTTAAACGGGGAGTGA
CTTGGGATGGGGG (SEQ ID NO. 202)
 contained AAAA at R¹, an arbitrary 30 nucleotide sequence at R², and no nucleotides at R³. This generated 35.7% as much light as SEQ ID NO. 25.

The next deoxyribozyme in the series 
GGAAGAAAAAGAATATCCCCCGACCACCAACCCAAGTAGACATTATGAGTGGGGAGTGA
CTTGGGATGGGGG (SEQ ID NO. 203)
 contained AAAA at R¹, an arbitrary 30 nucleotide sequence at R², and no nucleotides at R³. This generated 38.1% as much light as SEQ ID NO. 25.

The next deoxyribozyme in the series 
GGAAGAAAAAGAATATCCCCTGGCTCCCTCCAAGGGTAGGCATTCACGAGGGGGAGTGA
CTTGGGATGGGGG (SEQ ID NO. 204)
 contained AAAA at R¹, an arbitrary 30 nucleotide sequence at R², and no nucleotides at R³. This generated 41% as much light as SEQ ID NO. 25.

The next deoxyribozyme in the series 
GGAAGAAAAAGAATATCCCCGCAGTGTAGTGAGAGGACCTCAGCACACGCGGGGAGTGA
CTTGGGATGGGGG (SEQ ID NO. 205)
 contained AAAA at R¹, an arbitrary 30 nucleotide sequence at R², and no nucleotides at R³. This generated 106.2% as much light as SEQ ID NO. 25.

The next deoxyribozyme in the series GGAAGAAAAAGAATATCCCCAAAAGGGGAGTGACTTGGGATGGGGGGAT (SEQ ID NO. 206)
contained AAAA at R¹, AAAA at R², and an arbitrary 3 nucleotide sequence at R³. This generated 131% as much light as SEQ ID NO. 25.

The next deoxyribozyme in the series GGAAGAAAAAGAATATCCCCAAAAGGGGAGTGACTTGGGATGGGGGTGG (SEQ ID NO. 207)
contained AAAA at R¹, AAAA at R², and an arbitrary 3 nucleotide sequence at R³. This generated 90.4% as much light as SEQ ID NO. 25.

The next deoxyribozyme in the series GGAAGAAAAAGAATATCCCCAAAAGGGGAGTGACTTGGGATGGGGGTGT (SEQ ID NO. 208)
contained AAAA at R¹, AAAA at R², and an arbitrary 3 nucleotide sequence at R³. This generated 86.3% as much light as SEQ ID NO. 25.

The next deoxyribozyme in the series GGAAGAAAAAGAATATCCCCAAAAGGGGAGTGACTTGGGATGGGGGGCG (SEQ ID NO. 209)
contained AAAA at R¹, AAAA at R², and an arbitrary 3 nucleotide sequence at R³. This generated 53.4% as much light as SEQ ID NO. 25.

The next deoxyribozyme in the series GGAAGAAAAAGAATATCCCCAAAAGGGGAGTGACTTGGGATGGGGGTGG (SEQ ID NO. 210)
contained AAAA at R¹, AAAA at R², and an arbitrary 3 nucleotide sequence at R³. This generated 61.1% as much light as SEQ ID NO. 25.The next deoxyribozyme in the series GGAAGAAAAAGAATATCCCCAAAAGGGGAGTGACTTGGGATGGGGGACCTAGGCAA (SEQ ID NO. 211)
contained AAAA at R¹, AAAA at R², and an arbitrary 10 nucleotide sequence at R³. This generated 66.9% as much light as SEQ ID NO. 25.

The next deoxyribozyme in the series GGAAGAAAAAGAATATCCCCAAAAGGGGAGTGACTTGGGATGGGGGTGTTCATCAA (SEQ ID NO. 212)
contained AAAA at R¹, AAAA at R², and an arbitrary 10 nucleotide sequence at R³. This generated 90.9% as much light as SEQ ID NO. 25.

The next deoxyribozyme in the series GGAAGAAAAAGAATATCCCCAAAAGGGGAGTGACTTGGGATGGGGGGATAGACTCT (SEQ ID NO. 213)
contained AAAA at R¹, AAAA at R², and an arbitrary 10 nucleotide sequence at R³. This generated 79.8% as much light as SEQ ID NO. 25.

The next deoxyribozyme in the series GGAAGAAAAAGAATATCCCCAAAAGGGGAGTGACTTGGGATGGGGGATACCCTTGT (SEQ ID NO. 214)
contained AAAA at R¹, AAAA at R², and an arbitrary 10 nucleotide sequence at R³. This generated 47% as much light as SEQ ID NO. 25.

The next deoxyribozyme in the series GGAAGAAAAAGAATATCCCCAAAAGGGGAGTGACTTGGGATGGGGGCGATCTGACG (SEQ ID NO. 215)
contained AAAA at R¹, AAAA at R², and an arbitrary 10 nucleotide sequence at R³. This generated 125.8% as much light as SEQ ID NO. 25.

The next deoxyribozyme in the series 
GGAAGAAAAAGAATATCCCCAAAAGGGGAGTGACTTGGGATGGGGGCGAAGTTGGCAA
ACATTATAAGTTGGGGAG (SEQ ID NO. 216)
 contained AAAA at R¹, AAAA at R², and an arbitrary 30 nucleotide sequence at R³. This generated 49.5% as much light as SEQ ID NO. 25.

The next deoxyribozyme in the series 
GGAAGAAAAAGAATATCCCCAAAAGGGGAGTGACTTGGGATGGGGGCAAGGACTTTATA
CAATGCTCGCGGGTCGA (SEQ ID NO. 217)
 contained AAAA at R¹, AAAA at R², and an arbitrary 30 nucleotide sequence at R³. This generated 76.2% as much light as SEQ ID NO. 25.

The next deoxyribozyme in the series 
GGAAGAAAAAGAATATCCCCAAAAGGGGAGTGACTTGGGATGGGGGTGGAGCATGCATT
CTTGGACAAAATCTTGT (SEQ ID NO. 218)
 contained AAAA at R¹, AAAA at R², and an arbitrary 30 nucleotide sequence at R³. This generated 61% as much light as SEQ ID NO. 25.

The next deoxyribozyme in the series 
GGAAGAAAAAGAATATCCCCAAAAGGGGAGTGACTTGGGATGGGGGGTGCTACTTAAAT
TCTGTGTGTGACTTTAA (SEQ ID NO. 219)
 contained AAAA at R¹, AAAA at R², and an arbitrary 30 nucleotide sequence at R³. This generated 49.6% as much light as SEQ ID NO. 25.

The next deoxyribozyme in the series 
GGAAGAAAAAGAATATCCCCAAAAGGGGAGTGACTTGGGATGGGGGTTGGCTGCCGTCC
CAGGCGGCACGATAGAC (SEQ ID NO. 220)
 contained AAAA at R¹, AAAA at R², and an arbitrary 30 nucleotide sequence at R³. This generated 88.7% as much light as SEQ ID NO. 25.

The next deoxyribozyme in the series GGAAGATCCTGAATATCCCCTTGAGGGGAGTGACTTGGGATGGGGGCTAG (SEQ ID NO. 221)
contained an arbitrary 4 nucleotide sequence at R¹, R², and R³. This generated 252.4% as much light as SEQ ID NO. 25.

The next deoxyribozyme in the series GGAAGAGAACGAATATCCCCACCTGGGGAGTGACTTGGGATGGGGGCGAA (SEQ ID NO. 222)
contained an arbitrary 4 nucleotide sequence at R¹, R², and R³. This generated 59.9% as much light as SEQ ID NO. 25.

The next deoxyribozyme in the series GGAAGATGCCGAATATCCCCGTCGGGGGAGTGACTTGGGATGGGGGTACG (SEQ ID NO. 223)
contained an arbitrary 4 nucleotide sequence at R¹, R², and R³. This generated 78.7% as much light as SEQ ID NO. 25.

The next deoxyribozyme in the series GGAAGAGCGAGAATATCCCCACAGGGGGAGTGACTTGGGATGGGGGACGG (SEQ ID NO. 224)
contained an arbitrary 4 nucleotide sequence at R¹, R², and R³. This generated 66.1% as much light as SEQ ID NO. 25.

The next deoxyribozyme in the series GGAAGACCCTGAATATCCCCCCGTGGGGAGTGACTTGGGATGGGGGGCTC (SEQ ID NO. 225)
contained an arbitrary 4 nucleotide sequence at R¹, R², and R³. This generated 48.7% as much light as SEQ ID NO. 25.

The next deoxyribozyme in the series 
GGAAGACTTTACCTACGAATATCCCCGTAGGCACTCGGGGAGTGACTTGGGATGGGGGA
CGCTCCCAT (SEQ ID NO. 226)
 contained an arbitrary 10 nucleotide sequence at R¹, R², and R³. This generated 28.2% as much light as SEQ ID NO. 25.

The next deoxyribozyme in the series 
GGAAGATCGATGAGGCGAATATCCCCAGATCACCTAGGGGAGTGACTTGGGATGGGGGA
GCGGCACCG (SEQ ID NO. 227)
 contained an arbitrary 10 nucleotide sequence at R¹, R², and R³. This generated 40.7% as much light as SEQ ID NO. 25.

The next deoxyribozyme in the series 
GGAAGACTCCTTTTATGAATATCCCCCTGTCTTTAAGGGGAGTGACTTGGGATGGGGGCA
GATCGCTA (SEQ ID NO. 228)
 contained an arbitrary 10 nucleotide sequence at R¹, R², and R³. This generated 17.2% as much light as SEQ ID NO. 25.

The next deoxyribozyme in the series 
GGAAGATCGACCGCCAGAATATCCCCTCAGCGCGAGGGGGAGTGACTTGGGATGGGGGA
GAAGGAAGT (SEQ ID NO. 229)
 contained an arbitrary 10 nucleotide sequence at R¹, R², and R³. This generated 15% as much light as SEQ ID NO. 25.

The next deoxyribozyme in the series 
GGAAGATAACGTAGGGGAATATCCCCCCAGAAGGTAGGGGAGTGACTTGGGATGGGGGC
CCCGGACGT (SEQ ID NO. 230)
 contained an arbitrary 10 nucleotide sequence at R¹, R², and R³. This generated 19.8% as much light as SEQ ID NO. 25.

### Example 15: Method for detection of specific oligonucleotide sequences using a light-producing deoxyribozyme sensor

To generate a version of this deoxyribozyme that can sense oligonucleotides with specific sequences, we inserted part of the sequence of the target oligonucleotide into R², and the reverse complement of the full target oligonucleotide into R³. In the absence of the target, R² and R³ form a helix by Watson-Crick interactions that inhibits the deoxyribozyme. In the presence of the target, however, the inhibitory helix cannot form because R³ binds to the target rather than R². This results in catalytic activity and the production of light. Deoxyribozymes were tested as described in Example 4 (except using 0.25 mM rather than 1 mM CDP-Star^{™} substrate) in the presence of either 0 µM or 10 µM oligonucleotide ligand (= target).

The first sensor we generated GGAAGAAAAAGAATATCCCCCAATCGTGCGGGGAGTGACTTGGGATGGGGGAAGCACGA TTGACCTTC (SEQ ID NO. 231) (target is GAAGGTCAATCGTGC, SEQ ID NO. 232) generated light with a rate enhancement of 38.4-fold in the presence of 10 µM target and 1.9-fold in the absence of the target.

The second sensor we generated GGAAGAAAAAGAATATCCCCCTCTTAAGAGGGGAGTGACTTGGGATGGGGGAATCTTAA GAGAAGGGA (SEQ ID NO. 233) (target is TCCCTTCTCTTAAGA, SEQ ID NO. 234) generated light with a rate enhancement of 15-fold in the presence of 10 µM target and 2.7-fold in the absence of the target.

The third sensor we generated GGAAGAAAAAGAATATCCCCGGCACTGATGGGGAGTGACTTGGGATGGGGGAAATCAGT GCCCAGTGG (SEQ ID NO. 235) (target is CCACTGGGCACTGAT, SEQ ID NO. 236) generated light with a rate enhancement of 35.6-fold relative to the reaction in the absence of deoxyribozyme in the presence of 10 µM target and 2.4-fold in the absence of the target.

The fourth sensor we generated GGAAGAAAAAGAATATCCCCATGATCGGAGGGGAGTGACTTGGGATGGGGGAATCCGAT CATCCGAAG (SEQ ID NO. 237) (target is CTTCGGATGATCGGA, SEQ ID NO. 238) generated light with a rate enhancement of 44.8-fold relative to the reaction in the absence of deoxyribozyme in the presence of 10 µM target and 6.8-fold in the absence of the target.

The fifth sensor we generated GGAAGAAAAAGAATATCCCCAGTAATAGCGGGGAGTGACTTGGGATGGGGGAAGCTATT ACTTATCTT (SEQ ID NO. 239) (target is AAGATAAGTAATAGC, SEQ ID NO. 240) generated light with a rate enhancement of 37.5-fold relative to the reaction in the absence of deoxyribozyme in the presence of 10 µM target and 4.6-fold in the absence of the target.

## Claims

1. A deoxyribozyme containing or consisting of nucleotide sequence: 
5′-X¹X²X³AX⁴X⁵-R¹-X⁶X⁷X⁸X⁹X¹⁰-bp1-helix1-R²-helix2-bp2-X¹¹TGACX¹²TGGGAX¹³-helix3-X¹⁴-
R³-3′ (SEQ ID NO. 1)
 wherein
X¹ is G or T; X² is G or T; X³ is A or C; X⁴ is G or T; X⁵ is A or T; X⁶ is G or T or A; X⁷ is A or C or G or T; X⁸ is A or G or T; X⁹ is T or C or A or G; X¹⁰ is A or T or G; X¹¹ is G or A; X¹² is T or C or G; X¹³ is T or G or C; X¹⁴ is G or A or C or T;
R¹ represents a nucleotide sequence containing 3 to 30 nucleotides;
R² represents a nucleotide sequence containing 3 to 30 nucleotides;
R³ represents a nucleotide sequence containing 0 to 30 nucleotides;
bp 1 and bp2 are nucleotides selected from G, A, C, T, which together form a base pair;
helix1, helix2, and helix3 each independently denotes a nucleotide sequence preferably containing 4 or more nucleotides, wherein helix1, helix2, and helix3 together form a triple helical structure.

2. The deoxyribozyme according to claim 1, containing or consisting of nucleotide sequence: 
5′-X¹X²X³AX⁴X⁵-R¹-X⁶X⁷X⁸X⁹X¹⁰-bp1-helix1-R²-helix2-bp2-X¹¹TGACX¹²TGGGAX¹³-helix3-X¹⁴-
R³-3′ (SEQ ID NO. 1)
 wherein
X¹ is G; X² is G; X³ is A or C; X⁴ is G; X⁵ is A or T; X⁶ is G; X⁷ is A; X⁸ is A; X⁹ is T or C; X¹⁰ is A; X¹¹ is G; X¹² is T or C; X¹³ is T; X¹⁴ is G or A or C or T;
and wherein one or more, preferably one or two, of X¹, X², X⁴, X⁶-X¹³, are replaced by the following nucleotides: X¹ = T; X² = T; X⁴ = T; X⁶ = T or A; X⁷ = C or G or T; X⁸ = G or T; X⁹ = A or G; X¹⁰ = T or G; X¹¹ = A; X¹² = G; X¹³ = G or C.

3. The deoxyribozyme according to claim 1, containing or consisting of nucleotide sequence: 5'-GGX³AGX⁵-R¹-GAAX⁹A-bp1-helix1-R²-helix2-bp2-GTGACX¹²TGGGAT-helix3-X¹⁴-R³-3' (SEQ ID NO. 2) wherein
X³ is A or C; X⁵ is A or T; X⁹ is T or C; X¹² is Tor C; X¹⁴ is G or A or C or T.

4. The deoxyribozyme according to claim 1, containing or consisting of nucleotide sequence: 5'-GGAAGA-R¹-GAATA-bp1-helix1-R²-helix2-bp2-GTGACTTGGGAT-helix3-G-R³-3' (SEQ ID NO. 3), wherein
R¹ represents a nucleotide sequence containing 3 to 30 nucleotides;
R² represents a nucleotide sequence containing 3 to 30 nucleotides;
R³ represents a nucleotide sequence containing 0 to 30 nucleotides;
bp1 and bp2 are nucleotides selected from G, A, C, T, which together form a base pair;
helix1, helix2, and helix3 each independently denotes a nucleotide sequence preferably containing 4 or more nucleotides, wherein helix1, helix2, and helix3 together form a triple helical structure.

5. The deoxyribozyme according to any one of claims 1 to 4, wherein R³ is or contains an aptamer, and R² is or contains a sequence complementary to the aptamer.

6. The deoxyribozyme according to any one of claims 1 to 5, wherein helix1, helix2, helix3 is CCCC, GGGG, GGGG; or CCCT, AGGG, GGGT; or CTCC, GGAG, GAGG; or CCCT, AGGG, GGGA.

7. The deoxyribozyme according to claim 1, containing or consisting of a sequence selected from the group consisting of SEQ ID NO:8, SEQ ID NO:15, SEQ ID NO:16, SEQ ID NO: 17, SEQ ID NO:18, SEQ ID NO:19, SEQ ID NO. 20, SEQ ID NO. 21, SEQ ID NO:22, SEQ ID NO. 23, SEQ ID NO:24, SEQ ID NO:25, SEQ ID NO:26, SEQ ID NO:28, SEQ ID NO:29, SEQ ID NO:32, SEQ ID NO:34, SEQ ID NO:35, SEQ ID NO:36, SEQ ID NO:37, SEQ ID NO:38, SEQ ID NO:40, SEQ ID NO:41, SEQ ID NO:42. SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:76, SEQ ID NO:77, SEQ ID NO:78, SEQ ID NO:79, SEQ ID NO:80, SEQ ID NO:81, SEQ ID NO:82, SEQ ID NO:84, SEQ ID NO:92, SEQ ID NO:93, SEQ ID NO:94, SEQ ID NO:95, SEQ ID NO:96, SEQ ID NO:97, SEQ ID NO:98, SEQ ID NO:100, SEQ ID NO:101, SEQ ID NO:102, SEQ ID NO:108, SEQ ID NO:109, SEQ ID NO:110, SEQ ID NO:111, SEQ ID NO:113, SEQ ID NO:114, SEQ ID NO:115, SEQ ID NO:118, SEQ ID NO:119, SEQ ID NO:120, SEQ ID NO:121, SEQ ID NO:122, SEQ ID NO:123, SEQ ID NO:124, SEQ ID NO:125, SEQ ID NO:126, SEQ ID NO:127, SEQ ID NO:128, SEQ ID NO:129, SEQ ID NO:130, SEQ ID NO:131, SEQ ID NO. 132, SEQ ID NO:133, SEQ ID NO:134, SEQ ID NO:136, SEQ ID NO:143, SEQ ID NO:144, SEQ ID NO:145, SEQ ID NO:146, SEQ ID NO:148, SEQ ID NO:149, SEQ ID NO:152, SEQ ID NO:153, SEQ ID NO:154, SEQ ID NO:155, SEQ ID NO:156, SEQ ID NO:157, SEQ ID NO:158, SEQ ID NO:159, SEQ ID N0:160, SEQ ID NO:161, SEQ ID NO:162, SEQ ID NO:163, SEQ ID NO:164, SEQ ID NO:167, SEQ ID NO:168, SEQ ID NO:169, SEQ ID NO:170, SEQ ID NO:171, SEQ ID NO:172, SEQ ID NO:173, SEQ ID NO:174, SEQ ID NO:175, SEQ ID NO:176, SEQ ID NO:177, SEQ ID NO:178, SEQ ID NO:179, SEQ ID NO:180, SEQ ID NO:181, SEQ ID NO:182, SEQ ID NO:183, SEQ ID NO:184, SEQ ID NO:185, SEQ ID NO:186, SEQ ID NO:187, SEQ ID NO:188, SEQ ID NO:189, SEQ ID N0:190, SEQ ID N0:191, SEQ ID NO:192, SEQ ID NO:193, SEQ ID NO:194, SEQ ID NO:195, SEQ ID NO:196, SEQ ID NO:197, SEQ ID NO:198, SEQ ID NO:199, SEQ ID NO:200, SEQ ID NO:201, SEQ ID NO:202, SEQ ID NO:203, SEQ ID NO:204, SEQ ID NO:205, SEQ ID NO:206, SEQ ID NO:207, SEQ ID NO:208, SEQ ID NO:209, SEQ ID NO:210, SEQ ID NO:211, SEQ ID NO:212, SEQ ID NO:213, SEQ ID NO:214, SEQ ID NO:215, SEQ ID NO:216, SEQ ID NO:217, SEQ ID NO:218, SEQ ID NO:219, SEQ ID NO:220, SEQ ID NO:221, SEQ ID NO:222, SEQ ID NO:223, SEQ ID NO:224, SEQ ID NO:225, SEQ ID NO:226, SEQ ID NO:227, SEQ ID NO:228, SEQ ID NO:229, SEQ ID NO:230, SEQ ID NO:231, SEQ ID NO:233, SEQ ID NO:235, SEQ ID NO:237, and SEQ ID NO:239.

8. In vitro use of the deoxyribozyme according to any one of claims 1 to 7 as a signaling component of a molecular device in biosensing, diagnostics, and/or molecular computing.

9. A method of *in vitro* detection of a chemiluminescent substrate, containing a phosphate functional group, in a sample, containing the steps of:
- bringing the sample into contact with the deoxyribozyme according to any one of claims 1 to 7;
- causing the generation of an optical signal by cleavage of the phosphate functional group from the substrate present in the sample by self-phosphorylation of the deoxyribozyme;
- detecting the generated optical signal.

10. A method of *in vitro* detection of the deoxyribozyme according to any one of claims 1 to 7 in a sample, containing the steps of:
- bringing the sample into contact with a chemiluminescent substrate containing a phosphate functional group;
- causing the generation of an optical signal by cleavage of the phosphate functional group from the substrate by self-phosphorylation of the deoxyribozyme present in the sample;
- detecting the generated optical signal.

11. A method of *in vitro* detection of a ligand in a sample, containing the steps of:
- bringing the sample into contact with the deoxyribozyme according to any one of claims 1 to 7 and with a chemiluminescent substrate containing a phosphate functional group and capable of generating an optical signal upon cleavage of the phosphate functional group; wherein the deoxyribozyme is in an inactive state and wherein R³ contains an aptamer for the ligand so that when the ligand binds to the aptamer, the deoxyribozyme is converted into an active state;
- causing the generation of an optical signal by cleavage of the phosphate functional group from the substrate present in the sample by self-phosphorylation of the deoxyribozyme in the active state;
- detecting the generated optical signal.

12. The method according to claim 10, wherein the ligand is selected from an oligonucleotide, a protein, a small molecule, a metal ion, a lipid.

13. The method according to any one of claims 9-12, wherein the chemiluminescent substrate is a compound selected from the group of phosphate-stabilized 1,2-dioxetanes, preferably selected from disodium 2-chloro-5-(4-methoxyspiro{1,2-dioxetane-3,2 ' -(5' -chloro)tricyclo [3.3.1.1^{3.7}]decan}-4-yl)phenyl phosphate and disodium 3-(4-methoxyspiro {l,2-dioxetane-3,2'-(5'-chloro)tricyclo[3.3.1.13.7]decan}-4-yl) phenyl phosphate.

## Patentansprüche

1. Desoxyribozym, enthaltend oder bestehend aus der Nukleotidsequenz: 
5′-X¹X²X³AX⁴X⁵-R¹-X⁶X⁷X⁸X⁹X¹⁰-bp1-helix1-R²-helix2-bp2-X¹¹TGACX¹²TGGGAX¹³-helix3-X¹⁴-
R³-3′ (SEQ ID NO. 1)
 worin
X¹ ist G oder T; X² ist G oder T; X³ ist A oder C; X⁴ ist G oder T; X⁵ ist A oder T; X⁶ ist G oder T oder A; X⁷ ist A oder C oder G oder T; X⁸ ist A oder G oder T; X⁹ ist T oder C oder A oder G; X¹⁰ ist A oder T oder G; X¹¹ ist G oder A; X¹² ist T oder C oder G; X¹³ ist T oder G oder C; X¹⁴ ist G oder A oder C oder T;
R¹ ist eine Nukleotidsequenz, die 3 bis 30 Nukleotide enthält;
R² ist eine Nukleotidsequenz, die 3 bis 30 Nukleotide enthält;
R³ ist eine Nukleotidsequenz, die 0 bis 30 Nukleotide enthält;
bp 1 und bp2 sind Nukleotide, ausgewählt aus G, A, C, T, die zusammen ein Basenpaar bilden;
Helix1, Helix2 und Helix3 bezeichnen jeweils unabhängig voneinander eine Nukleotidsequenz, die vorzugsweise 4 oder mehr Nukleotide enthält, wobei Helix1, Helix2 und Helix3 zusammen eine dreifach helikale Struktur bilden.

2. Desoxyribozym nach Anspruch 1, enthaltend oder bestehend aus der Nukleotidsequenz: 
5′-X¹X²X³AX⁴X⁵-R¹-X⁶X⁷X⁸X⁹X¹⁰-bp1-helix1-R²-helix2-bp2-X¹¹TGACX¹²TGGGAX¹³-helix3-X¹⁴-
R³-3′ (SEQ ID NO. 1)
 worin
X¹ ist G; X² ist G; X³ ist A oder C; X⁴ ist G; X⁵ ist A oder T; X⁶ ist G; X⁷ ist A; X⁸ ist A; X⁹ ist T oder C; X¹⁰ ist A; X¹¹ ist G; X¹² ist T oder C; X¹³ ist T; X¹⁴ ist G oder A oder C oder T;
und wobei ein oder mehrere, vorzugsweise ein oder zwei, von X¹, X², X⁴, X⁶-X¹³, durch die folgenden Nukleotide ersetzt sind: X¹ = T; X² = T; X⁴ = T; X⁶ = T oder A; X⁷ = C oder G oder T; X⁸ = G oder T; X⁹ = A oder G; X¹⁰ = T oder G; X¹¹ = A; X¹² = G; X¹³ = G oder C.

3. Desoxyribozym nach Anspruch 1, enthaltend oder bestehend aus der Nukleotidsequenz: 5'-GGX³AGX⁵-R¹-GAAX⁹A-bpl-helixl-R²-helix2-bp2-GTGACX¹²TGGGAT-helix3-X¹⁴-R³-3' (SEQ ID NO. 2)
worin
X³ ist A oder C; X⁵ ist A oder T; X⁹ ist T oder C; X¹² ist T oder C; X¹⁴ ist G oder A oder C oder T.

4. Desoxyribozym nach Anspruch 1, enthaltend oder bestehend aus der Nukleotidsequenz: 5'-GGAAGA-R¹-GAATA-bpl-helixl-R²-helix2-bp2-GTGACTTGGGAT-helix3-G-R³-3' (SEQ ID NO. 3), worin
R¹ ist eine Nukleotidsequenz, die 3 bis 30 Nukleotide enthält;
R² ist eine Nukleotidsequenz, die 3 bis 30 Nukleotide enthält;
R³ ist eine Nukleotidsequenz, die 0 bis 30 Nukleotide enthält;
bp1 und bp2 sind Nukleotide, ausgewählt aus G, A, C, T, die zusammen ein Basenpaar bilden;
Helix1, Helix2 und Helix3 bezeichnen jeweils unabhängig voneinander eine Nukleotidsequenz, die vorzugsweise 4 oder mehr Nukleotide enthält, wobei Helix1, Helix2 und Helix3 zusammen eine dreifach helikale Struktur bilden..

5. Desoxyribozym nach einem der Ansprüche 1 bis 4, wobei R³ ist oder enthält ein Aptamer und R2 ist oder enthält eine zum Aptamer komplementäre Sequenz.

6. Desoxyribozym nach einem der Ansprüche 1 bis 5, wobei Helix1, Helix2, Helix3 sind CCCC, GGGG, GGGG; oder CCCT, AGGG, GGGT; oder CTCC, GGAG, GAGG; oder CCCT, AGGG, GGGA.

7. Desoxyribozym nach Anspruch 1, enthaltend oder bestehend aus der Nukleotidsequenz ausgewählt aus der Gruppe bestehend aus SEQ ID NO:8, SEQ ID NO:15. SEQ ID NO:16, SEQ ID NO: 17, SEQ ID NO:18, SEQ ID NO:19, SEQ ID NO. 20, SEQ ID NO. 21, SEQ ID NO:22, SEQ ID NO. 23, SEQ ID NO:24, SEQ ID NO:25, SEQ ID NO:26, SEQ ID NO:28, SEQ ID NO:29, SEQ ID NO:32, SEQ ID NO:34, SEQ ID NO:35, SEQ ID NO:36, SEQ ID NO:37, SEQ ID NO:38, SEQ ID NO:40, SEQ ID NO:41, SEQ ID NO:42. SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:76, SEQ ID NO:77, SEQ ID NO:78, SEQ ID NO:79, SEQ ID NO:80, SEQ ID NO:81, SEQ ID NO:82, SEQ ID NO:84, SEQ ID NO:92, SEQ ID NO:93, SEQ ID NO:94, SEQ ID NO:95, SEQ ID NO:96, SEQ ID NO:97, SEQ ID NO:98, SEQ ID NO:100, SEQ ID NO: 101, SEQ ID NO:102, SEQ ID NO:108, SEQ ID NO:109, SEQ ID NO: 110, SEQ ID NO:111, SEQ ID NO:113, SEQ ID NO:114 SEQ ID NO:115, SEQ ID NO: 118, SEQ ID NO:119 SEQ ID NO:120, SEQ ID NO:121 SEQ ID NO:122 SEQ ID NO:123, SEQ ID NO:124, SEQ ID NO:125, SEQ ID NO:126. SEQ ID NO:127, SEQ ID NO:128, SEQ ID NO: 129, SEQ ID NO:130, SEQ ID NO:131, SEQ ID NO. 132, SEQ ID NO:131, SEQ ID NO:134, SEQ ID NO:136, SEQ ID NO:143, SEQ ID NO:144, SEQ ID NO: 145, SEQ ID NO:146, SEQ ID NO:148, SEQ ID NO:149, SEQ ID NO:152, SEQ ID NO:153, SEQ ID NO:154, SEQ ID NO:155, SEQ ID NO:156, SEQ ID NO:157, SEQ ID NO:58, SEQ ID NO:159, SEQ ID NO:160, SEQ ID NO:161, SEQ ID NO:162, SEQ ID NO:163, SEQ ID NO:164, SEQ ID NO:167, SEQ ID NO:168, SEQ ID NO:169, SEQ ID NO:170, SEQ ID N0:171 _ SEQ ID NO:172, SEQ ID NO:173, SEQ ID NO:174, SEQ ID NO:175, SEQ ID NO:176, SEQ ID NO: 177, SEQ ID NO:178, SEQ ID NO:179, SEQ ID NO:180, SEQ ID N0:181, SEQ ID NO:182, SEQ ID NO:183, SEQ ID NO:184, SEQ ID NO:185, SEQ ID NO:186, SEQ ID NO:187,SEQ ID NO: 188, SEQ ID NO:189, SEQ ID NO:190, SEQ ID NO: 191, SEQ ID NO:192, SEQ ID NO:193, SEQ ID NO:194, SEQ ID NO:195, SEQ ID NO:196, SEQ ID NO:197, SEQ ID NO:198, SEQ ID N0:199, SEQ ID NO:200, SEQ ID NO:201, SEQ ID NO:202, SEQ ID NO:203, SEQ ID NO:204, SEQ ID NO:205, SEQ ID NO:206, SEQ ID NO:207, SEQ ID NO:208, SEQ ID NO:209, SEQ ID NO:210, SEQ ID NO:211, SEQ ID NO:212, SEQ ID NO:213, SEQ ID NO:214, SEQ ID NO:215, SEQ ID NO:216, SEQ ID NO:217, SEQ ID NO:218, SEQ ID NO:219, SEQ ID NO:220, SEQ ID NO:221, SEQ ID NO:222, SEQ ID NO:223, SEQ ID NO:224, SEQ ID NO:225, SEQ ID NO:226, SEQ ID NO:227, SEQ ID NO:228, SEQ ID NO:229, SEQ ID NO:230, SEQ ID NO:231, SEQ ID NO:233, SEQ ID NO:235, SEQ ID NO:237, und SEQ ID NO:239.

8. *In-vitro-*Verwendung des Desoxyribozyms nach einem der Ansprüche 1 bis 7 als Signalkomponente eines molekularen Geräts in der Biosensorik, Diagnostik und/oder molekularen Datenverarbeitung.

9. Verfahren zum *In-vitro-*Nachweis eines chemilumineszierenden Substrats, das eine funktionelle Phosphatgruppe enthält, in einer Probe, umfassend die Schritte:
- Inkontaktbringen der Probe mit dem Desoxyribozym nach einem der Ansprüche 1 bis 7;
- Bewirken der Erzeugung eines optischen Signals durch Abspaltung der funktionellen Phosphatgruppe vom in der Probe vorhandenen Substrat durch Selbstphosphorylierung des Desoxyribozyms;
- Erfassen des erzeugten optischen Signals.

10. Verfahren zum *In-vitro-*Nachweis des Desoxyribozyms nach einem der Ansprüche 1 bis 7 in einer Probe, umfassend die Schritte:
- Inkontaktbringen der Probe mit einem chemilumineszierenden Substrat, das eine funktionelle Phosphatgruppe enthält;
- Bewirken der Erzeugung eines optischen Signals durch Abspaltung der funktionellen Phosphatgruppe vom Substrat durch Selbstphosphorylierung des in der Probe vorhandenen Desoxyribozyms;
- Erfassen des erzeugten optischen Signals.

11. Verfahren zum *In-vitro-*Nachweis eines Liganden in einer Probe, umfassend die Schritte:
- Inkontaktbringen der Probe mit dem Desoxyribozym nach einem der Ansprüche 1 bis 7 und mit einem chemilumineszierenden Substrat, das eine funktionelle Phosphatgruppe enthält und in der Lage ist, bei Spaltung der funktionellen Phosphatgruppe ein optisches Signal zu erzeugen; wobei sich das Desoxyribozym in einem inaktiven Zustand befindet und wobei R³ ein Aptamer für den Liganden enthält, sodass das Desoxyribozym in einen aktiven Zustand umgewandelt wird, wenn der Ligand an das Aptamer bindet;
- Bewirken der Erzeugung eines optischen Signals durch Abspaltung der funktionellen Phosphatgruppe von dem in der Probe vorhandenen Substrat durch Selbstphosphorylierung des Desoxyribozyms im aktiven Zustand;
- Erfassen des erzeugten optischen Signals.

12. Verfahren nach Anspruch 11, wobei der Ligand ausgewählt ist aus einem Oligonukleotid, einem Protein, einem kleinen Molekül, einem Metallion, einem Lipid.

13. Verfahren nach einem der Ansprüche 9-12, wobei das chemilumineszierende Substrat eine Verbindung ist, ausgewählt aus der Gruppe der phosphatstabilisierten 1,2-Dioxetane, vorzugsweise ausgewählt aus Dinatrium-2-chlor-5-(4-methoxyspiro{1,2-dioxetan-3,2'-(5'-chlor)tricyclo[3.3.1.1^{3.7}]decan}-4-yl)phenylphosphat und Dinatrium-3-(4-methoxyspiro{1,2-dioxetan-3,2'-(5'-chlor)tricyclo[3.3.1.1^{3.7}]decan}-4-yl)phenylphosphat.

## Revendications

1. Un désoxyribozyme contenant ou constitué d'une séquence nucléotidique: 
5′-X¹X²X³AX⁴X⁵-R¹-X⁶X⁷X⁸X⁹X¹⁰-bp1-helix1-R²-helix2-bp2-X¹¹TGACX¹²TGGGAX¹³-helix3-X¹⁴-
R³-3′ (SEQ ID NO. 1)
 où
X¹ est G ou T; X² est G ou T; X³ est A ou C; X⁴ est G ou T; X⁵ est A ou T; X⁶ est G ou T ou A; X⁷ est A ou C ou G ou T; X⁸ est A ou G ou T; X⁹ est T ou C ou A ou G; X¹⁰ est A ou T ou G; X¹¹ est G ou A; X¹² est T ou C ou G; X¹³ est T ou G ou C; X¹⁴ est G ou A ou C ou T;
R¹ représente une séquence nucléotidique contenant 3 à 30 nucléotides;
R² représente une séquence nucléotidique contenant 3 à 30 nucléotides;
R³ représente une séquence nucléotidique contenant 0 à 30 nucléotides;
bp1 et bp2 sont des nucléotides choisis parmi G, A, C, T, qui forment ensemble une paire de bases;
helix1, helix2 et helix3 désignent chacun indépendamment une séquence nucléotidique contenant de préférence 4 nucléotides ou plus, où helix1, helix2 et helix3 forment ensemble une structure de triple hélice.

2. Désoxyribozyme selon la revendication 1, contenant ou constitué d'une séquence nucléotidique: 
5′-X¹X²X³AX⁴X⁵-R¹-X⁶X⁷X⁸X⁹X¹⁰-bp1-helix1-R²-helix2-bp2-X¹¹TGACX¹²TGGGAX¹³-helix3-X¹⁴-
R³-3′ (SEQ ID NO. 1)
 où
X¹ est G; X² est G; X³ est A ou C; X⁴ est G; X⁵ est A ou T; X⁶ est G; X⁷ est A; X⁸ est A; X⁹ est T ou C; X¹⁰ est A; X¹¹ est G; X¹² est T ou C; X¹³ est T; X¹⁴ est G ou A ou C ou T;
et où un ou plusieurs, de préférence un ou deux, parmi X¹, X², X⁴, X⁶-X¹³, sont remplacés par les nucléotides suivants: X¹ = T; X² = T; X⁴ = T; X⁶ = T ou A; X⁷ = C ou G ou T; X⁸ = G ou T; X⁹ = A ou G; X¹⁰ = T ou G; X¹¹ = A; X¹² = G; X¹³ = G ou C.

3. Désoxyribozyme selon la revendication 1, contenant ou constitué d'une séquence nucléotidique: 5'-GGX³AGX⁵-R¹-GAAX⁹A-bpl-helixl-R²-helix2-bp2-GTGACX¹²TGGGAT-helix3-X¹⁴-R3-3' (SEQ ID NO. 2)
où
X³ est A ou C; X⁵ est A ou T; X⁹ est T ou C; X¹² est T ou C; X¹⁴ est G ou A ou C ou T.

4. Désoxyribozyme selon la revendication 1, contenant ou constitué d'une séquence nucléotidique: 5'-GGAAGA-R¹⁻GAATA-bp1-helix1-R²-helix2-bp2-GTGACTTGGGAT-helix3-G-R³-3' (SEQ ID NO. 3), où
R¹ représente une séquence nucléotidique contenant 3 à 30 nucléotides;
R² représente une séquence nucléotidique contenant 3 à 30 nucléotides;
R³ représente une séquence nucléotidique contenant 0 à 30 nucléotides;
bp1 et bp2 sont des nucléotides choisis parmi G, A, C, T, qui forment ensemble une paire de bases;
helix1, helix2 et helix3 désignent chacun indépendamment une séquence nucléotidique contenant de préférence 4 nucléotides ou plus, où helix1, helix2 et helix3 forment ensemble une structure de triple hélice.

5. Désoxyribozyme selon l'une quelconque des revendications 1 à 4, où R³ est ou contient un aptamère, et R² est ou contient une séquence complémentaire de l'aptamère.

6. Désoxyribozyme selon l'une quelconque des revendications 1 à 5, où helix1, helix2, helix3 sont CCCC, GGGG, GGGG; ou CCCT, AGGG, GGGT; ou CTCC, GGAG, GAGG; ou CCCT, AGGG, GGGA.

7. Désoxyribozyme selon la revendication 1, contenant ou constitué d'une séquence sélectionnée dans le groupe constitué de SEQ ID NO:8, SEQ ID NO:15, SEQ ID NO:16, SEQ ID NO: 17, SEQ ID NO:18, SEQ ID N0:19, SEQ ID NO. 20, SEQ ID NO.21, SEQ ID NO:22, SEQ ID NO.23, SEQ ID NO:24, SEQ ID NO:25, SEQ ID NO:26, SEQ ID NO:28, SEQ ID NO:29, SEQ ID NO:32, SEQ ID NO:34, SEQ ID NO:35, SEQ ID NO:36, SEQ ID NO:37, SEQ ID NO:38, SEQ ID NO:40, SEQ ID NO:41, SEQ ID NO:42. SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:76, SEQ ID NO:77, SEQ ID NO:78, SEQ ID NO:79, SEQ ID NO:80, SEQ ID NO:81, SEQ ID NO:82, SEQ ID NO:84, SEQ ID NO:92, SEQ ID NO:93, SEQ ID NO:94, SEQ ID NO:95, SEQ ID NO:96, SEQ ID NO:97, SEQ ID NO:98, SEQ ID NO:100, SEQ ID NO:101, SEQ ID NO:102, SEQ ID NO: 108, SEQ ID NO:109, SEQ ID NO:110, SEQ ID NO:111, SEQ ID NO:113, SEQ ID NO: 114, SEQ ID NO: 115, SEQ ID NO: 118, SEQ ID NO:119, SEQ ID NO:120, SEQ ID NO:121, SEQ ID NO:122, SEQ ID NO:123, SEQ ID NO:124, SEQ ID NO:125, SEQ ID NO:126, SEQ ID NO:127, SEQ ID NO:128, SEQ ID NO:129, SEQ ID NO:130, SEQ ID NO:131, SEQ ID NO. 132, SEQ ID NO: 133, SEQ ID NO:134, SEQ ID NO:136, SEQ ID NO:143, SEQ ID NO:144, SEQ ID NO:145, SEQ ID NO: 146, SEQ ID NO:148, SEQ ID NO:149, SEQ ID NO:152, SEQ ID NO:153, SEQ ID NO:154, SEQ ID NO:155, SEQ ID NO:156, SEQ ID NO:157, SEQ ID NO:158, SEQ ID NO:159, SEQ ID NO: 160, SEQ ID NO: 161, SEQ ID NO: 162, SEQ ID NO:163, SEQ ID NO: 164, SEQ ID NO:167, SEQ ID NO:168, SEQ ID NO:169, SEQ ID NO:170, SEQ ID NO:171, SEQ ID NO:172, SEQ ID NO:173, SEQ ID NO:174, SEQ ID NO:175, SEQ ID NO:176, SEQ ID NO:177, SEQ ID NO:178, SEQ ID NO:179, SEQ ID NO:180, SEQ ID NO:181, SEQ ID NO:182, SEQ ID NO:183, SEQ ID NO:184, SEQ ID NO:185, SEQ ID NO:186, SEQ ID NO:187,SEQ ID NO:188, SEQ ID NO:189, SEQ ID NO:190. SEQ ID NO:191, SEQ ID NO:192, SEQ ID NO: 193, SEQ ID NO:194, SEQ ID NO:195. SEQ ID NO:196, SEQ ID NO:197, SEQ ID NO:198, SEQ ID NO:199, SEQ ID NO:200, SEQ ID NO:201, SEQ ID NO:202, SEQ ID NO:203, SEQ ID NO:204, SEQ ID NO:205, SEQ ID NO:206, SEQ ID NO:207, SEQ ID NO:208, SEQ ID NO:209, SEQ ID NO:210, SEQ ID NO:211, SEQ ID NO:212, SEQ ID NO:213, SEQ ID NO:214, SEQ ID NO:215, SEQ ID NO:216, SEQ ID NO:217, SEQ ID NO:218, SEQ ID NO:219, SEQ ID NO:220, SEQ ID NO:221, SEQ ID NO:222, SEQ ID NO:223, SEQ ID NO:224, SEQ ID NO:225, SEQ ID NO:226, SEQ ID NO:227, SEQ ID NO:228, SEQ ID NO:229, SEQ ID NO:230, SEQ ID NO:231 , SEQ ID NO:233, SEQ ID NO:235, SEQ ID NO:237, et SEQ ID NO:239.

8. Utilisation *in vitro* du désoxyribozyme selon l'une quelconque des revendications 1 à 7 comme composant de signalisation d'un dispositif moléculaire en biodétection, diagnostic et/ou informatique moléculaire.

9. Procédé de détection *in vitro* d'un substrat chimioluminescent, contenant une fonction phosphate, dans un échantillon, comprenant les étapes de:
- mise en contact de l'échantillon avec le désoxyribozyme selon l'une quelconque des revendications 1 à 7;
- provoquer la génération d'un signal optique par clivage de la fonction phosphate du substrat présent dans l'échantillon par auto-phosphorylation du désoxyribozyme;
- détecter le signal optique généré.

10. Procédé de détection *in vitro* du désoxyribozyme selon l'une quelconque des revendications 1 à 7 dans un échantillon, comprenant les étapes de:
- mise en contact de l'échantillon avec un substrat chimioluminescent contenant une fonction phosphate;
- provoquer la génération d'un signal optique par clivage de la fonction phosphate du substrat par autophosphorylation du désoxyribozyme présent dans l'échantillon;
- détecter le signal optique généré.

11. Procédé de détection *in vitro* d'un ligand dans un échantillon, contenant les étapes suivantes:
- mise en contact de l'échantillon avec le désoxyribozyme selon l'une quelconque des revendications 1 à 7 et avec un substrat chimioluminescent contenant une fonction phosphate et capable de générer un signal optique lors de la clivage de la fonction phosphate; le désoxyribozyme étant dans un état inactif et R³ contenant un aptamère pour le ligand de sorte que lorsque le ligand se lie à l'aptamère, le désoxyribozyme soit converti dans un état actif;
- provoquer la génération d'un signal optique par clivage de la fonction phosphate du substrat présent dans l'échantillon par auto-phosphorylation du désoxyribozyme à l'état actif ;
- détecter le signal optique généré.

12. Procédé selon la revendication 11, où le ligand est choisi parmi un oligonucléotide, une protéine, une petite molécule, un ion métallique, un lipide.

13. Procédé selon l'une quelconque des revendications 9 à 12, où le substrat chimioluminescent est un composé choisi dans le groupe des 1,2-dioxétanes stabilisés par phosphate, de préférence choisi parmi 2-chloro-5-(4-méthoxyspiro}1,2-dioxétane-3,2'-(5'-chloro)tricyclo[3.3.1.1^{3.7}]décan}-4-yl)phénylphosphate disodique et 3-(4-méthoxyspiro{1,2-dioxétane-3,2'-(5'-chloro)tricyclo[3.3.1.1^{3.7}]décan}-4-yl)phénylphosphate disodique.
